(19) Europäisches Patentamt
European Patent Office
Office européen des brevets

(11) Publication number: **0 088 695 B1**

(12) # EUROPEAN PATENT SPECIFICATION

(45) Date of publication of patent specification: **03.06.92**

(51) Int. Cl.⁵: **G01N 33/53**, A61K 47/00, A61K 39/395, A61K 39/44, G01N 33/574, G01N 33/58, A61K 49/00, A61K 45/05

(21) Application number: **83400461.6**

(22) Date of filing: **07.03.83**

The file contains technical information submitted after the application was filed and not included in this specification

(54) **Antibody conjugates.**

(30) Priority: **09.03.82 US 356315**
**16.11.82 US 442050**

(43) Date of publication of application:
**14.09.83 Bulletin 83/37**

(45) Publication of the grant of the patent:
**03.06.92 Bulletin 92/23**

(84) Designated Contracting States:
**BE CH DE FR GB IT LI LU NL SE**

(56) References cited:
**EP-A- 44 167**
**EP-A- 56 322**
**DE-A- 2 921 781**
**FR-A- 2 418 237**
**GB-A- 2 013 688**
**JP-A-79 155 094**
**EP-A- 55 575**
**DE-A- 2 651 680**
**FR-A- 2 312 259**
**GB-A- 1 446 536**
**JP-A-77 154 520**
**US-A- 4 279 992**

(73) Proprietor: **CYTOGEN CORPORATION**
**201 College Road East Forrestal Research Center**
**Princeton New Jersey 08540(US)**

(72) Inventor: **McKearn, Thomas Joseph**
**RD 1, Box 22-4**
**New Hope, PA 18938(US)**
Inventor: **Goers, John Walter Frank**
**38 Sayre Drive**
**Princeton, NJ 08540(US)**
Inventor: **Rodwell, John Dennis**
**430 Ramsey Road**
**Yardley, PA 19067(US)**
Inventor: **Lee, Chyi**
**1347 Ute Road**
**North Brunswick, NJ 08902(US)**

(74) Representative: **Warcoin, Jacques et al**
**Cabinet Régimbeau 26, avenue Kléber**
**F-75116 Paris(FR)**

**EP 0 088 695 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

### 1. FIELD OF THE INVENTION

The present invention relates to the covalent attachment of antibodies, including monoclonal antibodies and polyclonal antibodies (conventional antisera), to soluble conjugate partners (compounds or linkers) to form antibody conjugates which are useful in a variety of diagnostic and therapeutic applications.

More particularly, the invention is directed to methods of attachment to the carbohydrate moieties of an antibody molecule located outside the antigen binding domain of the antibody molecule. The product conjugates substantially retain the entire immunospecificity and immunoreactivity of the antibodies from which they are made, and further retain the ability to activate complement.

This invention also relates to the general area of carrier systems capable of delivering compounds to target sites in vivo. Such systems include the general area of the delivery of pharmaceutical agents or other compounds to target sites in vivo, and in vivo imaging systems (e.g., tumor imaging systems), based upon antigen-antibody interactions.

The present invention also includes the attachment of a substrate-linker to an antibody so that the resulting antibody conjugates retain the ability to bind antigen and activate complement. In selected applications, this promotes the release of the compound in its active form at the target site.

### 2. BACKGROUND OF THE INVENTION

#### 2.1. COVALENT ATTACHMENT

Various reactions can be used to covalently attach compounds to antibodies. This has been accomplished by reaction of the amino acid residues of the antibody molecule, including the amine groups of lysine, the free carboxylic acid groups of glutamic and aspartic acid, the sulfhydryl groups of cysteine and the various moieties of the aromatic amino acids.

There are serious disadvantages to these methods of covalent attachment to the polypeptide backbone of an antibody molecule. The amino acid sequences of the light and heavy chains of immunoglobulins contain all of the amino acids relatively regularly and randomly dispersed throughout the molecule, including the antigen binding region. To the extent any chemical modification occurs in this antigen binding region, one has introduced a change in the recognition element of the antibody. Such changes would be expected to, and, in fact do, change the affinity and specificity of the antibody for antigen. In a population of different antibodies, such alteration in the antigen binding region results in complete inactivation of some antibodies and in lesser degrees of inactivation of others in relation to the proximity of the alteration to the antigen binding site. This inactivation may be due to a change within the antigen binding site to change the conformation of the binding site so as to make it unreactive, or may be due to a change in a region outside the antigen binding region so as to limit access of antigen to the antigen binding region.

Probably the most commonly used non-specific method of covalent attachment is the carbodiimide reaction to link carboxy groups of a compound to amino groups of the antibody. Additionally, bifunctional agents such as dialdehydes or imidoesters have been used to link the amino group of a compound to amino groups of the antibody molecule. Some investigators have used the Schiff base reaction to link compounds to antibody molecules. This method involves the periodate oxidation of a drug or cytotoxic agent that contains a glycol or hydroxy group, thus forming an aldehyde which is then reacted with the antibody molecule. Attachment occurs via formation of a Schiff base with amino groups of the antibody molecule. Additionally, compounds with reactive sulfhydryl groups have been coupled to antibody molecules. Isothiocyanate can be used as a coupling agent for covalently attaching compounds to antibodies. This method has been used to attach fluorescent compounds to antibody molecules for use in fluorescence microscopy (Brandtzaeg, 1973, Scand. J. Immunol. 2:273-290) and cell sorting systems (Loken and Herzenberg, 1975, Annals N.Y. Acad. Sci. 254:163-171).

Interchain disulfide bonds can also be used as sites of covalent attachment. If one is successful in selectively reducing only the interchain disulfide bonds, several functional properties of the antibody may be adversely affected, such as functional affinity, agglutination ability and the ability to fix complement.

#### 2.2. NON-COVALENT ATTACHMENT

Alternative methods of attachment to antibody molecules outside the antigen binding region (outside the variable domains) may involve use of antibodies directed against the constant domain of the antibody

molecule, or use of Staphylococcal protein A which is known to bind specifically to a site on the constant region. Since these modes of attachment are non-covalent, either approach is limited and would not be efficient for separation and purification schemes. Any dissociation of antigen would likely lead to release of antibody from the support to which it is non-covalently attached, so selective elution of antigen cannot be accomplished. Additionally, with respect to carrier systems (see Section 2.3), since non-covalent bonds are more likely to be broken before the antibody complex reaches the target site, covalent linkages are preferred.

## 2.3 CARRIER SYSTEMS

A number of agents have been utilized as carrier molecules with limited success in drug delivery systems. In practice the carrier should be non-toxic and target site specific. Ideally there should be a mechanism for release of the active form of the compound from the carrier at the target site. Carrier molecules such as DNA, liposomes, proteins, steroid hormones, and antibodies (whole antibody molecules or Fab fragments) have been used in conjunction with a broad spectrum of pharmaceutical or cytotoxic agents such as: radioactive compounds (e.g., $I^{125}$, $I^{131}$); agents which bind DNA, for instance, alkylating agents or various antibiotics (e.g., daunomycin, adriamycin, chlorambucil); antimetabolites such as methotrexate; agents which act on cell surfaces (e.g., venom phospholipases and microbial toxins); and protein synthesis inhibitors (e.g., diphtheria toxin and toxic plant proteins). For reviews on the subject see Bale et al., 1980, Cancer Research 40:2965-2972; Ghose and Blair, 1978, J. Natl. Cancer Inst. 61(3):657-676; Gregoriadis, 1977, Nature 265:407-411; Gregoriadis, 1980, Pharmac. Ther. 10:103-118; Trouet et al., 1980, Recent Results Cancer Res. 75:229-235. Some of the delivery systems which are more pertinent to the present invention are discussed below.

Liposome mediated delivery of pharmaceutical agents has major drawbacks because of its lack of target specificity. Recently, investigators have attempted to overcome this problem by covalently attaching whole antibody or Fab fragments to liposomes containing a pharmaceutical agent (Heath et al., 1981, Biochim. Biophys. Acta 640:66-81; Huang et al., 1980, J. Biol. Chem. 255(17):8015-8018; Jansons and Mallet, 1981, Anal. Biochem. 111:54-59, Martin et al., 1981, Biochem. 20:4229-4238). Others have reported the coupling of protein A (Staph A protein) to liposomes in order to direct the preparation to multiple specific targets which have previously been bound to antibodies. Such targets are simply limited by the antibodies used (Leserman et al., 1980, Nature 288:602-604). However, an intrinsic problem of particular importance in any lipsome carrier system is that in most cases the targeted liposome does not selectively reach its target site in vivo. Whether or not liposomes are coated with antibody molecules, liposomes are readily phagocytosed by macrophages and removed from circulation before reaching other target sites.

Most investigators have recognized another major problem inherent in the liposome targeting system, which is that binding of the targeted liposomes to the target cell does not ensure incorporation of the liposome contents, hence, the pharmaceutical agent, into the target cell (Weinstein et al., 1978, Biochim. Biophys. Acta 509:272-288). A few investigators have tried to overcome this problem by targeting liposomes using receptor specific compounds which would be internalized into the target cell (Leserman et al., 1980, Proc. Natl. Acad. Sci., U.S.A. 77(7):4089-4093; Mauk et al., 1980, Proc. Natl. Acad. Sci., U.S.A. 77(8): 4430-4434). The problem of internalizing the liposome contents still exists, however, because not all tumor cells are actively phagocytic. For instance, fibrosarcoma cells are much less phagocytic than are cells of lymphomas and leukemias. Thus, these liposome mediated delivery systems rely upon the ability of the target cell itself to internalize a substance which will be ultimately lethal to the cell.

Finally, if the liposome is internalized into the target cell, there is no assurance that the pharmaceutical agent will be released in its active form. After phagocytosis the liposome contents are packaged within lysosomes of the target cell. The array of proteolytic enzymes contained within the lysosome may either degrade the pharmaceutical agent or render the agent inactive by altering its structure or cleaving the active site. The variety of proteolytic enzymes contained in the lysosome makes it very difficult, if not impossible, to devise bonding arrangements that will allow release of the pharmaceutical agent in its active form. Thus, reliance upon the enzyme content of the target cell lysosomes is, at best, a haphazard system to effect release of the active form of the pharmaceutical agent.

A number of investigators have reported target systems involving attachment of compounds or pharmaceutical agents directly to conventional antibodies, monoclonal antibodies, or to Fab portions of antibodies directed against tumor antigens. See review articles, supra, and Blythman et al., 1981, Nature 290:145-146; Davis and Preston, 1981, Science 213:1385-1388; Hurwitz et al., 1979, Int. J. Cancer 24:461-470); U.S. Patent No. 4,093,607; and U.K. Patent No. 1446536. Urdal and Hakomori (1980, J. Biol. Chem. 255(21):10509-10579) describe an antibody targeted, avidin mediated, drug killing of tumor cells.

Various investigators have examined proteins other than antibodies as carriers in a target system. For example, desialylated glycoproteins are preferentially taken up by hepatocytes. See review articles, supra, and Bernstein et al., 1978, J. Natl. Cancer Inst. 60(2):379-384.

Although antibody carrier systems are more specific for the target than are the liposome carrier systems, a significant problem exists in the release of the pharmaceutical agent at the target site. As in the liposome mediated systems, the antibody-drug compounds must be internalized by the tumor cell so that the drug can be released through cleavage by lysosomal enzymes (see review articles, supra). Additionally, the nonspecific linkage of the pharmaceutical agent to random sites on the antibody molecule may interfere with antigen binding capacity, thus reducing the effectiveness of the system.

Radiopharmaceutical techniques currently used in non-invasive in vivo imaging methods are based upon the ability of the target organ to remove the radiopharmaceutical label from the circulation. These techniques utilize various substances to deliver radioactive compounds to desired targets; such substances include substrates, substrate analogs, ligands, hormones, radionuclides, bifunctional chelates (linker groups containing a chelator at one end which is able to bind a heavy metal or radionuclide and a reactive group at the other end which can covalently attach to a target cell) and liposomes (Spencer, R.P., ed. 1980. Radiopharmaceuticals Structure-Activity Relationship. Grune & Stratton, New York; Eckelman and Levanson, 1977, Intl. J. Appl. Radiation and Isotoper 28:67-82). Other non-invasive techniques currently available are emission tomography, nuclear magnetic resonance imaging, and in vivo spectroscopy. See review article by Brownell et al., 1982, Science 215:619-626 where the authors suggest the application of labeled antibodies in the field of radiopharmaceuticals.

## 2.4. CITED PRIOR ART

FR-A-2 418 237 discloses a process for obtaining compounds based on glycoproteins, usable as insolubilized glycoproteins or as markers. DE-A-2 651 680 describes a method for attaching an amino-containing solid, insoluble support to a carbohydrate residue of an organic compound. GB-A-2 013 688 describes insolubilized compositions of antibodies or antigens coupled to a water-insoluble substrate. EP-A-0 044 167 discloses conjugates in which the attachment between the antibody and a cytotoxic agent is via a disulfide bond.

JP 155 094/79 and JP 154 520/77 disclose antibodies tagged with a compound or fixed to an insoluble support suited to in vitro uses.

## 3. SUMMARY OF THE INVENTION

The present invention relates to the covalent attachment of compounds to antibody molecules so that the resulting antibody conjugates retain the ability to bind antigen and activate complement. In particular, such methods of attachment consist in attachment to the carbohydrate moieties of antibodies.

In a prefered embodiment, the present invention is concerned with covalent attachment to the carbohydrate moieties of the heavy chains of antibodies, and, generally, to the $CH_2$ domain. Since the carbohydrate is the constant region of the antibody (outside the variable domain), modification of the carbohydrate per se will not directly introduce reactive or interfering groups into the variable domain. Hence, such an approach is an attractive means for covalent modification of antibodies without seriously adversely affecting immunoreactivity and immunospecificity.

According to the preferred embodiment, antibodies are modified by covalent attachment to the carbohydrate moiety using known reagents and reactions. Instead of dealing with protein chemistry, the present applicants utilize those reactions of carbohydrate chemistry unique to carbohydrates to modify the carbohydrate of the glycoproteins. In principle, there are may different enzymatic and non-enzymatic reactions that are directed primarily, if not exclusively, to carbohydrate moieties.

The object of the invention is a soluble antibody conjugate for a medical use comprising: a water-soluble therapeutic or diagnostic compound containing an amine group selected from the group consisting of secondary amine, hydrazine, hydrazide, hydroxylamine, phenylhydrazine and semicarbazide, attached through a covalent bond to an aldehyde group of an oxidized carbohydrate moiety of an antibody or antibody fragment said covalent bond between said amine group and said aldehyde group being an enamine, hydrazone, oxime, phenylhydrazone, semicarbazone or a reduced form thereof, and said soluble antibody conjugate having (i) substantially the same immunoreactivity and immunospecificity as the unconjugated antibody or antibody fragment, and (ii) aqueous solubility, such that the conjugate is suitable for in vivo administration.

Another object is also a soluble antibody conjugate for a medical use, comprising: a water-soluble

therapeutic or diagnostic compound which comprises a water-soluble linker attached to a second compound containing an amine group selected from the group consisting of primary amine, secondary amine, hydrazine, hydrazide, hydroxylamine; phernylhydrazine and semicarbazide attached through a covalent bond to an aldehyde group of an oxidized carbohydrate moiety of an antibody or antibody fragment said covalent bond between said amine group and said aldehyde group being an imine, enamine, hydrazone, oxime, phenylhydrazone, semicarbazone or a reduced form thereof, and said soluble antibody conjugate having (i) substantially the same immunoreactivity and immunospecificity as the conjugated antibody or antibody fragment, and (ii) aqueous solubility such that the conjugate is suitable for in vivo administration.

The product conjugates of the present invention have been found to retain substantial, if not complete. immunoreactivity and immunospecificity. When such conjugates are prepared using monoclonal antibodies, the association constant and index of heterogeneity are unaffected by the present mode of covalent modification, whereas conventional means of modification decrease the average binding constant and introduce functional heterogeneity. Furthermore, the reagents usable with the novel techniques of the invention can be extremely selective so that there is no detectable reaction of the polypeptide portion of the antibody molecule. This procedure can be performed on intact molecules or functional monovalent fragments of antibodies. Finally, the modification can be done with a variety of antibody classes, including IgA, IgD, IgE, IgG and IgM, and antibodies from any source, including monoclonal antibodies.

This invention also encompasses the use of antibodies as carrier molecules for the targeting of a compound or compounds for delivery to specific cells, tissues, organs, or any other site in vivo, and the subsequent complement-mediated release of the compound at the target site. Alternatively, release may be mediated by serum proteases.

Antibodies directed against any desired target (e.g., antigenic determinants of tumor cells, virus, fungi bacteria, or parasites) may be used as carrier molecules. Although conventional antibodies may be used as carrier molecules, monoclonal antibodies offer the advantages of increased specificity for antigen, improved efficiency of the delivery system and ease in production.

Once administered in vivo, the carrier antibody molecule will attach to the antigenic determinant of the target site. The subsequent release of the linked compound is dependent upon complement activation or serum proteases. Complement is the collective name for a group of serum proteins which are sequentially activated (the complement cascade) by the formation of certain immune complexes. Several of the complement components of the cascade have proteolytic activity which is specific for particular substrates or chemical bonds.

According to one process of the present invention, a compound is attached to an antibody carrier molecule of an immunoglobulin class that is capable of complement activation. This attachment is accomplished via linkers which are susceptible to cleavage by one or more of the activated complement enzymes, and one or more different compounds may be attached to each antibody molecule. The resulting antibody conjugate is administered to an individual. Subsequent to the binding of the modified antibody and antigen in vivo, the individual's serum complement is activated and the compounds will be selectively cleaved and released at the target site. Such conjugates may also be used for the in vitro detection and identification of target antigen in a complement-fixation assay system.

For the practice of this invention it is desirable to attach the compound to the antibody molecule without interfering with either the antigen binding capacity of the antibody, or with the ability to activate complement (also called complement fixation). The present invention describes the novel linkers and methods of attachment which may be used to attach compounds to any antibody capable of activating complement.

Alternatively, certain techniques, such as tumor imaging, require that the compound remain attached to the target site. In an alternate embodiment, when cleavage at the target site is not desirable, then the linker group utilized is insensitive to the activated complement proteins, or the antibody molecule is of a class or type that does not activate complement.

Finally, for delivery of other compounds, e.g., hormones or neurotransmitters, where it may be desirable to cleave the compound without activation of the complement cascade, one may use a serum protease sensitive linker attached to an antibody that does not fix complement.

## 4. BRIEF DESCRIPTION OF FIGURES

The present invention may be more fully understood by reference to the following detailed description of the invention, examples of specific embodiments of the invention and the appended figures in which:

FIG. 1 is a schematic representation of an antibody molecule or immunoglobulin of the IgG class (a), and of the IgM class (b).

FIG. 2 represents a portion of the complement cascade activated by the classical pathway. C1 through

C9 represent complement proteins. The bar over certain numbers indicates an active enzyme. S' represents a site on the cell membrane.

FIG. 3 depicts a general reaction scheme for the attachment of the antineoplastic drug, Alkeran (Burroughs-Wellcome), to the peptide CBZ-gly-gly-arg.

FIG. 4 represents the excitation spectra for (a) unoxidized antibody and (b) antibody oxidized in accordance with Section 6.1.

FIG. 5 represents the excitation and emission spectra of the Phenylhydrazine-Tripeptide-AMC compound prepared in accordance with Section 6.2.

FIG. 6 represents the excitation and emission spectra of the Antibody-Phenylhydrazine-Tripeptide-AMC (APTA) conjugate prepared in accordance with Section 6.3.

FIG. 7 represents Sips plots of fluorescent quenching data using unmodified antibody

(●—●);

antibody modified by the method of the invention

(Δ—Δ);

and antibody modified by attachment (carbodiimide) to aspartic and/or glutamic amino acids

(□—□).

FIG. 8 represents the results of experiments showing the specific complement mediated release of AMC along with certain controls. Fluorescence is monitored at 460 nm with excitation at 380 nm. An increase in fluorescence indicates release of AMC from the Antibody-Phenylhydrazine-Tripeptide-AMC (APTA) conjugate; (a) represents APTA conjugate incubated with glutaraldehyde-fixed sheep red blood cells and human complement; (b) represents APTA conjugate incubated with glutaraldehyde-fixed rat red blood cells and human complement; (c) represents APTA conjugate incubated with glutaraldehyde-fixed sheep red blood cells; (d) represents APTA conjugate alone.

## 5. DETAILED DESCRIPTION OF THE INVENTION

Glycoproteins are one of the several types of biologically important macromolecules which have found application in selected therapeutic and/or diagnostic settings. Although the currently recognized classes of glycoproteins are far from complete, a catalog of such materials would include immunoglobulins, serum complement components, a variety of enzymes, cell surface histocompatibility antigens and cell surface receptors. These compounds have carbohydrate residues which are covalently attached to a polypeptide backbone. Antibodies are one class of glycoproteins whose carbohydrate moieties are generally located on the heavy chain of the immunoglobulin molecule. (See FIG. 1 for a schematic representation of several immunoglobulins.) The Fab or Fab' fragments of any immunoglobulins which contain carbohydrate moieties may also be utilized in the reaction scheme described herein. The Fab' fragments of IgG immunoglobulins are obtained by cleaving the antibody molecule with pepsin [resulting in a bivalent fragment, $(Fab')_2$] or with papain (resulting in two univalent fragments, 2 Fab).

### 5.1. CHOICE OF ANTIBODY

According to the present invention, antibodies directed against any antigen or hapten may be used. Although conventional antibodies (antisera) may be used, monoclonal antibodies offer several advantages. Each monoclonal antibody is specific for one antigenic determinant. In addition, unlimited amounts of each monoclonal antibody can be produced. Antibodies used in the present invention may be directed against any determinant, e.g., tumor, bacterial, fungal, viral, parasitic, mycoplasmal, histocompatibility, differentiation and other cell membrane antigens, pathogen surface antigens, toxins, enzymes, allergens, drugs and any biologically active molecules.

Drugs of particular interest are opioids, amphetamines, barbiturates, steroids, catecholamines, dilantin, theophylline, histamine, cannabinoids, and the like. For a more complete list of antigens, see U.S. Patent

4,193,983, particularly columns 7-11, which patent specification is incorporated herein by reference.

When delivery and release of the compound attached to the antibody are desired, immunoglobulins of the IgM class or certain subclasses of IgG should be used since these are the types of immunoglobulin that are known to activate complement. In other applications carrier immunoglobulins may be used which are not capable of complement activation. Such immunoglobulin carriers may include: certain classes of antibodies such as IgA, IgD, IgE; certain subclasses of IgG; or certain fragments of immunoglobulins, e.g., half antibody molecules (a single heavy:light chain pair), or Fab, Fab', or $(Fab')_2$ fragments. When imaging of in vivo targets is to be accomplished, the use of antibody fragments as carriers is advantageous since these fragments permeate target sites at an increased rate. Additionally, a combination of antibodies reactive to different antigenic determinants may be used.

TABLE I

DRUGS FOR ANTIBODY-MEDIATED DELIVERY

| TYPE | NAME/CLASS | LINKAGE | MANUFACTURER(S) |
|---|---|---|---|
| Antibacterials | | | |
| | Aminoglycosides | | |
| | Streptomycin | ester/amide | |
| | Neomycin | ester/amide | Dow, Lilly, Dome, Pfipharmics |
| | Kanamycin | ester/amide | Bristol |
| | Amikacin | ester | Bristol |
| | Gentamicin | ester/amide | Upjohn, Wyeth, Schering |
| | Tobramycin | ester/amide | Lilly |
| | Streptomycin B | ester/amide | Squibb |
| | Spectinomycin | ester | Upjohn |
| | Ampicillin | amide | Squibb, Parke-Davis, Comer, Wyeth, Upjohn, Bristol, SKF |
| | Sulfanilamide | amide | Merrell-National |
| | Polymyxin | amide | Burroughs-Wellcome, Dow, Parke-Davis |
| | Chloramphenicol | ester | Parke-Davis |
| Antivirals | | | |
| | Acyclovir | | Burroughs-Wellcome |
| | Vira A | ester/amide | Parke-Davis |
| | Symmetrel | amide | Endo |
| Antifungals | | | |
| | Nystatin | ester | Squibb, Primo, Lederle, Pfizer, Holland-Rantos |
| Antineoplastics | | | |
| | Adriamycin | ester/amide | Adria |
| | Cerubidine | ester/amide | Ives |
| | Bleomycin | ester/amide | Bristol |
| | Alkeran | amide | Burroughs-Wellcome |
| | Valban | ester | Lilly |
| | Oncovin | ester | Lilly |
| | Fluorouracil | ester | Adria, Roche, Herbert |
| Radiopharmaceuticals | $I^{125}$ | | |
| | $I^{131}$ | | |
| | $^{99m}Tc$ (Technetium) | | |
| | $In^{111}$ | | |
| Heavy Metals | | | |
| | Barium | | |
| | Gold | | |
| | Platinum | | |
| Antimycoplasmals | | | |
| | Tylosine | | |
| | Spectinomycin | | |

## 5.2. METHODS FOR ATTACHING COMPOUNDS TO ANTIBODY MOLECULES

In addition to the utilization of conventional methods, the present invention includes several novel methods for attaching compounds to the carbohydrate moieties of the antibody molecule. Whichever method is used, the attachment must not significantly change the essential characteristics of the antibody, such as immunospecificity and immunoreactivity. Additional considerations include simplicity of reaction

and stability of the antibody conjugate produced.

5.2.1. ATTACHMENT TO THE CARBOHYDRATE MOIETY OF THE ANTIBODY

As explained in detail below, the carbohydrate side chains of antibodies may be selectively oxidized to generate aldehydes. The resulting aldehydes may then be reacted with amine groups (e.g., ammonia derivatives such as hydroxylamine, hydrazine, phenylhydrazine, or semicarbazide) to form a Schiff base (e.g., oxime, hydrazone, phenylhydrazone or semicarbazone, respectively).

Alternatively, the carbohydrate moiety of the antibody may be modified by enzymatic techniques so as to enable attachment to or reaction with other chemical groups. One example of such an enzyme is galactose oxidase which oxidizes galactose in the presence of oxygen.

5.2.1.1. CHEMICAL METHODS OF OXIDATION

Oxidation of the carbohydrate portion or moiety of antibody molecules leads to formation of aldehyde groups. A variety of oxidizing agents can be used, such as periodic acid, paraperiodic acid, sodium metaperiodate and potassum metaperiodate. Among these, oxygen acids and salts thereof are preferred since secondary or undesirable side reactions are less frequent. For a general discussion, see Jackson, 1944, Organic Reactions 2, p. 341; Bunton, 1965, Oxidation in Organic Chemistry, Vol. 1 (Wiberg, ed.), Academic Press, New York, p. 367.

Oxidation of antibodies with these oxidizing agents can be carried out by known methods. In the oxidation, the antibody is used generally in the form of an aqueous solution, the concentration being generally less than 100 mg/mℓ, preferably 1 to 20 mg/mℓ. When an oxygen acid or a salt thereof is used as the oxidizing agent, it is used generally in the form of an aqueous solution, and the concentration is generally 0.001 to 10mM and preferably 1.0 to 50mM. The amount of the oxygen acid or salt thereof depends on the kind of antibody, but generally it is used in excess, for example, twice to ten times as much as the amount of the oxidizable carbohydrate. The optimal amount, however, can be determined by routine experimentation.

In the process for oxidizing antibodies with oxygen acids or salts thereof, the optional ranges include a pH of from about 4 to 8, a temperature of from 0° to 37°C, and a reaction period of from about 15 minutes to 12 hours.

During the oxidation of the glycoprotein with an oxygen acid or a salt thereof, light is preferably excluded to prevent over oxidation of the glycoprotein.

5.2.1.2. ENZYMATIC METHODS OF OXIDATION

Oxidation of the carbohydrate portion of antibody molecules may also be done with the enzyme, galactose oxidase [Cooper, et al., 1959, J. Biol. Chem. 234:445-448]. The antibody is used in aqueous solution, the concentration being generally 0.5-20 mg/mℓ. The enzyme generally is used at about 5-100 units per mℓ of solution, at a pH ranging from about 5.5 to about 8.0. The influence of pH, substrate concentration, buffers and buffer concentrations on enzyme reaction are reported in Cooper, et al., supra.

5.2.1.3. PREPARATION OF ANTIBODY CONJUGATES

The antibody conjugates of the invention may be produced by reacting the oxidized antibody with any suitable conjugate partner having an available amine group. The immediately resulting products (antibody conjugates) contain a carbon-nitrogen double bond resulting from elimination of a molecule of water from the initial addition products:

$$\text{Antibody-}\overset{H}{C}\text{=O} + NH_2\text{-R} \longrightarrow \text{Antibody-CH=N-R} + H_2O$$

For a general discussion of the reaction of aldehydes with hydrazides, see March, 1978, Advanced Organic Chemistry: Reactions, Mechanisms and Structure, McGraw-Hill Co., New York, p. 674.

A solution of the oxidized antibody at a concentration of from about 0.5 to 20 mg/mℓ is mixed with the conjugate partner (molar ratios of reactive conjugate partner group to antibody aldehyde ranging from about 1 to about 10,000) and the solution incubated for from about 1 to 18 hours. Suitable temperatures are from

0 to 37° C and pH may be from about 6 to 8.

5.2.1.4. STABILIZATION OF THE ANTIBODY CONJUGATES

After the antibody conjugates have been formed between the antibody and its conjugate partner as described in Section 5.2.1.3, the antibody conjugates can optionally be stabilized with a suitable reducing agent, such as sodium cyanoborohydride or sodium borohydride:

$$\text{Antibody-CH=N-R} \xrightarrow[\text{agent}]{\text{reducing}} \text{Antibody-CH}_2\text{-NH-R}$$

Reducing agent is generally added to a molar excess of from about 10 to 100 fold molar excess over available aldehyde groups. For a general discussion, see Jentoft and Dearborn, 1979, J. Biol. Chem. 254:4359.

5.3. CONJUGATE PARTNERS

According to the methods of the invention, antibodies can be attached to any suitable conjugate partner. The major limiting factor is that the attachment reaction must (1) be selective enough to limit competing, undesirable reactions and (2) be sufficiently mild so as not to severely interfere with antibody reactivity and selectivity. For purposes of description, conjugate partners are divided into (a) compounds of interest, which are attached directly to the antibody and (b) soluble linkers.

5.3.1. COMPOUNDS OF INTEREST

The conjugate partner to which the antibodies are attached may be any compound which retains its essential properties after reaction with the antibody, and which enables the antibody to substantially retain immunospecificty and immunoreactivity.

For example, when it is desired to attach an aldehyde of the oxidized carbohydrate portion of a glycoprotein to a conjugate compound, the compound should desirably contain a reactive group such as an amino or hydrazide group. Such conjugate compounds include various proteins, such as hormones, enzymes, transport proteins, catalysts, chelating compounds, receptor proteins and immunoglobulins and fluorescent or chemiluminescent compounds or potentially fluorescent or chemiluminescent compounds. By "potentially" fluorescent or chemiluminescent compounds is meant compounds which fluoresce or chemiluminesce only after reaction, modification or combination with another agent. Where a compound of interest does not contain an amino group, the compound can be modified to make an amino or hydrazide group available for coupling.

The compound to be attached to an antibody for use in a delivery system is selected according to the purpose of the intended application (e.g., killing, prevention of cell proliferation, hormone therapy, target imaging, or gene therapy). Such compounds may include, for example, pharmaceutical agents, toxins, fragments of toxins, alkylating agents, enzymes, antibiotics, antimetabolites, antiproliferative agents, hormones, neurotransmitters, DNA, radioopaque dyes, radioactive isotopes, fluorogenic compounds, marker compounds, lectins, compounds which alter cell membrane permeability. Table I lists some of the pharmaceutical agents that may be employed in the herein described invention and in no way is meant to be an exhaustive list. Finally, a combination of compounds may be used.

5.3.2. SOLUBLE LINKERS

According to the invention, antibodies may be covalently attached to any compound of interest through an intermediate soluble linking agent, or linker, having a number of reactive groups, one to react with antibody and one to react with the compound of interest or to an insoluble support. The linker must be chosen such that neither the reaction with antibody (or a compound of interest nor the final product adversely affect antibody reactivity and selectivity. In general, these linkers would include heterobifunctional linkers such as mercaptoethanolamine which would bond to the oxidized antibody via its amino group. After deblocking the sulfur atom, free sulfhydryl groups would be available for further reaction. The linker may be of any appropriate size to permit the distance between the antibody and the compound of interest to be

selected as desired.

## 5.4. CARRIER SYSTEMS

According to one embodiment of the present invention, a compound may be attached to an antibody directed against a target antigen. When release of the active form of the compound at the target site is desired, then the compound is attached to specific sites on an antibody molecule (immunoglobulin) belonging to a class that is capable of activating the complement cascade. This attachment is accomplished via chemical bonds (e.g., an ester or amide linkage) and linker groups (e.g., peptides or amino acids) which are susceptible to cleavage by one or more of the serum complement components.

The chemical linking methods described herein allow the resulting antibody conjugate to retain the ability to bind antigen and to activate the complement cascade. As a result, when the conjugate is administered to an individual, the subsequent formation of immune complexes with target antigens in vivo activates the individual's serum complement. If the linker is designed to be susceptible to cleavage by complement, the compound will be cleaved at the target site by one or more of the enzymes of the complement cascade. Since release of the compound occurs after delivery to the target site the efficiency of the target delivery system is greatly improved.

The method of the present invention offers another advantage over other targeting systems. It is known that all cells of a tumor do not each possess the target antigenic determinant. Thus, delivery systems which require internalization into the target cell will effect successful delivery only to those tumor cells that possess the antigenic determinant and that are capable of internalizing the complex. Tumor cells that do not possess the antigenic determinant or are incapable of internalization, will escape treatment.

According to the method of the present invention, antibody carrier molecules deliver the compound to the target cells. More importantly, however, once attached to the target cell, the method described in the present invention allows the release of the active compound by the individual's activated complement enzymes. Once released, the compound is then free to permeate the target sites, e.g., tumor mass. As a result, the compound will act on tumor cells that do not possess the antigenic determinant as well as those tumor cells that do possess the determinant. Additionally, the entire process is not dependant upon internalization of the conjugate.

The method of targeted delivery described herein may be employed for a number of purposes. The choice of antibodies, linkers, and compounds used to make the conjugates depends upon the purpose of delivery. The delivery and release of pharmaceutical compounds at specific target sites may result in selectively killing or preventing the proliferation of tumor cells, cancer cells, fungi, bacteria, parasites, or virus. The targeted delivery of hormones, enzymes, or neurotransmitters to selected sites may also be accomplished. Ultimately the method of the present invention may have an application in gene therapy programs wherein DNA or specific genes may be delivered in vivo or in vitro to target cells that are deficient in that particular gene.

In an alternate embodiment, the conjugate may be designed so that the compound is delivered to the target but not released. Thus, the present invention may also be used for locating, detecting, and quantitating specific sites in vivo such as tumors, organs, or sites of infection. This embodiment of the invention is particularly useful in imaging systems.

Specifically, in the case of imaging, a radiopharmaceutical or heavy metal is (a) covalently bound to the linker or (b) non-covalently bound to the linker via a chelator. Therefore, depending upon the nature of the target and purpose of delivery, a wide range of antibodies, linkers, and compounds of interest may be used in a variety of combinations.

## 5.5. SERUM COMPLEMENT AND SELECTION OF LINKERS

According to the method of the present invention, when release of a compound is desired, that compound is linked to a specific site on an antibody of the IgM class or certain subclasses of IgG (FIG. 1). The resulting conjugate retains the ability to bind antigen and activate the complement cascade.

Complement is the collective name for a group of serum proteins which can be activated in one of two ways, the classical pathway and the properdin pathway (Müller-Eberhard, Hospital Practice, August 1977:33-43). The classical pathway is initiated by the binding of antibodies of the IgM class or certain subclasses of IgG to its corresponding antigen whereas the properdin pathway is dependent upon the serum protein, properdin and other non-immunoglobulin serum factors (Reid and Porter, 1981, Ann. Rev. Biochem. 50:433-464).

The classical pathway is the pathway of particular importance for the practice of the present invention.

The classical pathway is characterized by the formation of certain antibody-antigen complexes (or immune complexes) which activate the proteolytic enzymes of the complement system (Borsos and Rapp, 1965, J. Immunol. 95:559-566; Cohen, 1968, J. Immunol. 100:407-413; Cohen and Becker, 1968, J. Immunol. 100:403-406; Ishizaka et al., 1968, J. Immunol. 100:1145-1153). These activated complement enzymes cleave and activate other components of the complement cascade (FIG. 2). Ultimately the formation of an "attack complex" (or lytic complex) is induced resulting in disruption of target cell membrane integrity.

The first component activated in the classical pathway is C1 which becomes a protease that acts on both C2 and C4. Activated C1 ($C\overline{1}$) has a specific esterase activity. Activated C4,2 ($C\overline{4_b,2_a}$), sometimes called C3 convertase, is a complex which proteolytically cleaves C3, and together with activated C3 ($C\overline{3b}$), cleaves C5. Cleavage of C3 is the first step in common between the classical and properdin pathways of complement activation.

The enzymatic activities of both $C\overline{1}$ and $C\overline{4_b,2_a}$ have been recently studied using model synthetic substrates (see Table II) which are cleaved at the carboxy terminal ester or amide bond in vitro. These synthetic substrates may be used as linkers between an antibody molecule and a compound as described in the present invention. Such linkers will allow for the specific complement mediated cleavage and subsequent release of the compound in its active form at the target site. However, any substrate which is susceptible to cleavage by any of the components of complement may be used as a linker.

Thus, according to the present invention, a compound is joined to one end of the substrate linker group and the other end of the linker group is attached to a specific site on the antibody molecule. For example, if the compound has an hydroxy group or an amino group, the compound may be attached to the carboxy terminus of a peptide, amino acid or other suitably chosen linker via an ester or amide bond, respectively. For example, such compounds may be attached to the linker peptide via a carbodiimide reaction. If the compound contains functional groups that would interfere with attachment to the linker, these interfering functional groups can be blocked before attachment of the compound and deblocked once the conjugate is made. For example, FIG. 3 depicts a general reaction scheme for the attachment of the antineoplastic drug, Alkeran (Burroughs-Wellcome) to the peptide CBZ-gly-gly-arg. The opposite or amino terminal of the linker group is then modified for binding to an antibody molecule which is capable of activating complement.

The compound may be attached to the linker before or after the linker is attached to the antibody molecule. In certain applications such as attachment of short-lived radioisotopes to antibodies, it is desirable to first produce a stable modified antibody as an intermediate wherein the linker is free of an associated compound. Depending upon the particular application, the compound may be covalently attached to the linker of the modified antibody. These peptide linkers may be variable in length since distance of the substrate from the antibody molecule may affect efficiency of cleavage which occurs at the amide or ester bond between the linker and the compound. These linkers may also include organic compounds, for example, organic polymers of any desired length, one end of which can be covalently attached to specific sites on the antibody molecule. The other end of the organic polymer may be attached to an amino acid or peptide linker. Table III lists other substrates that may be used as linker groups to prepare the antibody conjugates of the present invention. (In the table n may be an integer including zero.) These sequences were derived from those of the complement substrate sequences by substituting amino acids with similar acid-base properties. This list is not exhaustive.

Thus when these conjugates bind to antigen in the presence of complement the amide or ester bond which attaches the compound to the linker will be cleaved, resulting in release of the compound in its active form. These conjugates, when administered to an individual, will accomplish delivery and release of the compound at the target site, and are particularly effective for the in vivo delivery of pharmaceutical agents, antibiotics, antimetabolites, antiproliferative agents, and cytotoxins.

## TABLE II

### SYNTHETIC SUBSTRATES FOR COMPLEMENT COMPONENTS

| | Reference No.* |
|---|---|
| **For $C\overline{1}$:** | |
| N-Boc-tyrosine o-nitrophenyl ester | 1 |
| N-Boc-phenylalanine o-nitrophenyl ester | 1 |
| α-N-Boc-lysine o-nitrophenyl ester | 1 |
| N-CBZ-tyrosine p-nitrophenyl ester | 2 |
| **For $C\overline{4_b,2_a}$:** | |
| N-acetyl-gly-lys-methyl ester | 3 |
| α-N-CBZ-lys-methyl ester | 3 |
| α-N-acetyl-lys-methyl ester | 3 |
| Boc-leu-gly-arg-7-amino-4-methylcoumarin | 4 |

---

* 1. Sim, et al., 1977, Biochem. J. 163:219-27.
2. Bins, 1969, Biochemistry 8:4503-10.
3. Cooper, N.R., 1975, Biochemistry 14:4245-51.
4. Caporale, et al., 1981, J. Immunol. 128:1963-65.

## TABLE III

### LINKER GROUPS FOR ATTACHMENT OF COMPOUNDS OF INTEREST (CI) TO ANTIBODY MOLECULES[1]

A. Linkers For Cleavage by $C\overline{1}$

$$H_2N\text{-}(a.a.)_n\text{-}\left\{\begin{array}{l}\text{-lys-}\\ \text{-tyr-}\\ \text{-phe-}\\ \text{-arg-}\end{array}\right\}\text{-*-CI}$$

B. Tripeptide Sequences For Cleavage by $C\overline{4_{b},2_{a}}$

$$H_2N\text{-}(a.a.)_n\text{-}\left\{\begin{array}{l}\text{-leu-ala-arg-}\\ \text{-leu-ala-lys-}\\ \text{-leu-ala-tyr-}\\ \text{-leu-leu-arg-}\\ \text{-leu-leu-lys-}\\ \text{-leu-leu-tyr-}\\ \text{-leu-gly-arg-}\\ \text{-leu-gly-lys-}\\ \text{-leu-gly-tyr-}\\ \text{-leu-val-arg-}\\ \text{-leu-val-lys-}\\ \text{-leu-val-tyr-}\\ \text{-leu-ile-arg-}\\ \text{-leu-ile-lys-}\\ \text{-leu-ile-tyr-}\end{array}\right\}\text{-*-CI}$$

$$H_2N\text{-}(a.a.)_n\text{-}\left\{\begin{array}{l}\text{-ala-ala-arg-}\\ \text{-ala-ala-lys-}\\ \text{-ala-ala-tyr-}\\ \text{-ala-leu-arg-}\\ \text{-ala-leu-lys}\\ \text{-ala-leu-tyr-}\\ \text{-ala-gly-arg-}\\ \text{-ala-gly-lys-}\\ \text{-ala-gly-tyr-}\\ \text{-ala-val-arg-}\\ \text{-ala-val-lys-}\\ \text{-ala-val-tyr-}\\ \text{-ala-ile-arg-}\\ \text{-ala-ile-lys-}\\ \text{-ala-ile-tyr-}\end{array}\right\}\text{-*-CI}$$

III B. Tripeptide Sequences For Cleavage by $\overline{C4_{b},2_{a}}$ (Continued)

$$H_2N\text{-}(a.a.)_n\text{-}\left\{\begin{array}{l}\text{-gly-ala-arg-}\\ \text{-gly-ala-lys-}\\ \text{-gly-ala-tyr-}\\ \text{-gly-leu-arg-}\\ \text{-gly-leu-tyr-}\\ \text{-gly-leu-lys-}\\ \text{-gly-gly-arg-}\\ \text{-gly-gly-lys-}\\ \text{-gly-gly-tyr-}\\ \text{-gly-val-arg-}\\ \text{-gly-val-lys-}\\ \text{-gly-val-tyr-}\\ \text{-gly-ile-arg-}\\ \text{-gly-ile-lys-}\\ \text{-gly-ile-tyr-}\end{array}\right\}\text{-*-CI}$$

$$H_2N\text{-}(a.a.)_n\text{-}\left\{\begin{array}{l}\text{-val-ala-arg-}\\ \text{-val-ala-lys-}\\ \text{-val-ala-tyr-}\\ \text{-val-leu-arg-}\\ \text{-val-leu-lys-}\\ \text{-val-leu-tyr-}\\ \text{-val-gly-arg-}\\ \text{-val-gly-lys-}\\ \text{-val-gly-tyr-}\\ \text{-val-val-arg-}\\ \text{-val-val-lys-}\\ \text{-val-val-tyr-}\\ \text{-val-ile-arg-}\\ \text{-val-ile-lys-}\\ \text{-val-ile-tyr-}\end{array}\right\}\text{-*-CI}$$

$$H_2N(a.a.)_n\text{-}\left\{\begin{array}{l}\text{-ile-ala-arg-}\\ \text{-ile-ala-lys-}\\ \text{-ile-ala-tyr-}\\ \text{-ile-leu-arg-}\\ \text{-ile-leu-lys-}\\ \text{-ile-leu-tyr-}\\ \text{-ile-gly-arg-}\\ \text{-ile-gly-lys-}\\ \text{-ile-gly-tyr-}\\ \text{-ile-val-arg-}\\ \text{-ile-val-lys-}\\ \text{-ile-val-tyr-}\\ \text{-ile-ile-arg-}\\ \text{-ile-ile-lys-}\\ \text{-ile-ile-tyr-}\end{array}\right\}\text{-*-CI}$$

## III C. Peptide Sequences for Cleavage by $C\overline{4_{b},2_{a}}$

$H_2N$- { -leu-gly- / -leu-leu- / -leu-ala- / -leu-val- / -leu-ile- / -gly-gly- / -gly-leu- / -gly-ala- / -gly-val- / -gly-ile- / -ala-gly- / -ala-leu- / -ala-ala- / -ala-val- / -ala-ile- / -val-gly- / -val-leu- / -val-ala- / -val-val- / -val-ile- / -ile-gly- / -ile-leu- / -ile-ala- / -ile-val- / -ile-ile- } -Tripeptide[2]-*-CI

---

1 The asterisk (*) represents either an amide bond (Linker-C-NH-CI) or an ester bond (Linker-C-O-CI).

2 Tripeptide represents any of the Tripeptides listed in Table III B.

In certain applications, release of the compound is not desirable. Thus, under an alternate embodiment of the present invention a compound is attached to antibody molecules via linkers which are not susceptible to cleavage by complement enzymes. These linkers may include amino acids, peptides, or other organic compounds which may be modified to include functional groups that can subsequently be utilized in attachment to antibody molecules or antibody fragments by the methods described herein. A general formula for such an organic linker is

$W-(CH_2)_n-Q$

wherein
W is either $-NH-CH_2-$ or $-CH_2-$;
Q is an amino acid, peptide, chelator (e.g., diethylenetriaminepentaacetic acid) or chelator derivative ; and
n is an integer from 0 to 20.

Alternatively, a compound may be attached to antibody molecules or antibody fragments which do not activate complement. When using carrier antibodies that are incapable of complement activation, this attachment may be accomplished using linkers that are susceptible to cleavage by activated complement or using linkers that are not susceptible to cleavage by activated complement.

This non-cleavage approach is also particularly useful for making antibody conjugates for use in imaging systems for locating tumors, organs, sites of infection, etc., where release of the compound is not desirable. In such imaging systems, the use of antibody fragments offers a distinct advantage since such

fragments diffuse and permeate tissue masses more easily than do whole antibody molecules. In addition, antibody fragments do not cross placental barriers (Bellanti, 1978, Immunology II. W.B. Saunders, Philadelphia). Therefore, tumor imaging may be practiced in pregnant females.

In still another embodiment, it may be necessary to construct the linker in such a way as to optimize the spacing between the compound and the antibody. This may be accomplished by use of a linker of the general structure

$W-(CH_2)_n-Q$

wherein Q is an amino acid or peptide and W and n are as described above.

In yet another application of target delivery, a release of the compound without complement activation is desired since activation of the complement cascade will ultimately lyse the target cell. Hence, this approach is useful when delivery and release of the compound should be accomplished without killing the target cell. Such is the goal when delivery of cell mediators such as hormones, enzymes, corticosteroids, neurotransmitters, genes or enzymes to target cells is desired. These conjugates may be prepared by attaching the compound to an antibody molecule or fragment that is not capable of activating complement via a linker that is mildy susceptible to cleavage by serum proteases. When this conjugate is administered to an individual, antigen-antibody complexes will form quickly whereas cleavage of the compound will occur slowly, thus resulting in release of the compound at the target site.

The first steps in activation of the classical complement pathway require an interaction between C1 and antibody-antigen complexes. This interaction requires that a site in the Fc region of the antibody molecule be present (FIG. 1). Some of the carbohydrate moieties are located on the Fc region of the immunoglobulin which are required in order for C1 binding to occur. Removal of the carbohydrate moiety results in loss of the ability of the immune complex to bind component C1 of complement (Winkelhake et al., 1980, J. Biol. Chem. 255:2822-2828). The Fab or Fab' fragments of any immunoglobulins which contain carbohydrate moieties may be utilized in the reaction scheme described herein. An example of such an immunoglobulin is the human IgM sequenced by Putnam, et al. (1973, Science 132:287).

In accordance with one embodiment of the invention, the substrate linkers are modified by attaching hydrazine or hydrazide deriviatives to one end of the linker. The unmodified sites on the linker may or may not be covalently attached to a compound. For instance, the substrate linkers which are attached to a compound via an ester or amide link, as described in Section 5.5 (see Table II and Table III) are modified by attaching a hydrazide (e.g., phenylhydrazine) to the opposite amino terminus of the peptide chain. This results in the following structure (N.B., the symbol * signifies an amide or ester bond):

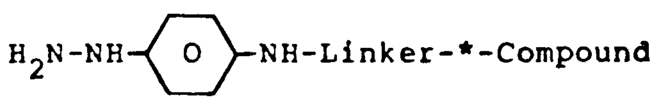

Although in the structure shown the hydrazine is in the para position, one could alternatively use compounds with the hydrazine moiety in the ortho or meta positions. These hydrazide derivatives of the peptide linkers which are attached to a compound via an ester or amide link are then reacted with an oxidized immunoglobulin, or immunoglobulin fragment containing an oxidized carbohydrate. This results in hydrazone formation and the covalent attachment of the compound to the carbohydrate side chain of the immunoglobulin via a linker group which is susceptible to cleavage by complement. If desired, the linker utilized may be resistant to cleavage by either activated complement or serum proteases (e.g. a linker which includes a chelator or chelator derivative for use in an imaging system). In another application the linker may be designed to be susceptible to cleavage by a serum protease. The covalent attachment of the linker to the carrier antibody as described herein does not interfere with the antigen binding site of the molecule nor with complement fixation. The resulting structure is schematically represented below (N.B., the symbol * signifies an amide or ester bond):

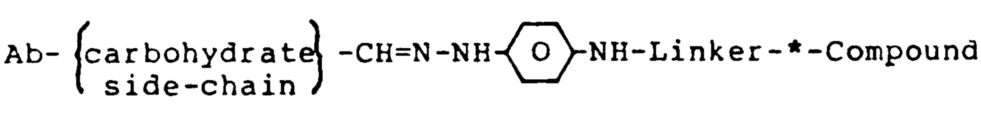

Although this section is directed primarily to reactions with the carbohydrate moieties of antibodies,

such techniques are applicable to other classes of glycoproteins as well.

## 5.6. ADDITIONAL USES OF ANTIBODY CONJUGATES

The antibody conjugates of the invention are useful for a variety of applications in medicine and industry, including diagnostics and therapeutics. All of these applications capitalize on the ability of antibodies to distinguish specifically between chemical compounds of slightly differing structure.

## 6. EXAMPLES: SERIES I

The following examples illustrate methods for the specific attachment of an antibody molecule to a compound of interest via an intermediate soluble linker.

## 6.1. OXIDATION OF THE CARBOHYDRATE MOIETY OF THE ANTIBODY MOLECULE

The antibody molecule used in this example was a monoclonal IgM (designated no. 171) specific for antigenic determinants on sheep red blood cells. To prepare the monoclonal antibody, Lewis rats were immunized with a single injection of sheep red blood cells. Three days later, spleen cells from the immunized rats were harvested and fused with the myeloma line SP2/0 Ag14 according to the method of McKearn, et al., 1979, Immunol. Rev. 47:91-115. Cloned cells were then grown and the resulting monoclonal antibody was purified as described by Klinman and McKearn, 1981, J. Immunol. Meth. 42:1-9.

Oxidation of the antibody carbohydrate moiety was accomplished by reacting the antibody with galactose oxidase by a modification of the method of Cooper, et al., supra. To this end, 3.8 mg of no. 171 monoclonal antibody was added to 1 mℓ of buffer consisting of 0.135M NaCl, 0.015M Tris-HCl (pH 7.0), 0.5mM $MgCl_2$, and 0.15mM $CaCl_2$. Subsequently, a 0.1 mℓ aliquot of a solution of galactose oxidase (Worthington Biochemical Co., Freehold, N.J.) at a concentration of 52 units of enzyme/mℓ of the same buffer was added to the antibody solution. Finally, 43 µg of catalase (Worthington Biochemical Co., Freehold, N.J.) dissolved in an additional 0.1 mℓ of the same buffer was added to the reaction mixture (the catalase was added to degrade hydrogen peroxide that is generated during the oxidation reaction). The reaction mixture was incubated for 48 hours at room temperature, then stored at 4 ° C. FIG. 4 represents the excitation spectra for unoxidized (a) and oxidized (b) antibodies.

## 6.2. PREPARATION OF THE TRIPEPTIDE-AMC FOR ATTACHMENT TO THE ANTIBODY MOLECULE

For the purposes of the present example, a synthetic fluorogenic compound was utilized as the conjugate partner. The properties of this synthetic compound are such that the bound and free states of the fluorogenic compound are spectrofluorometrically distinguishable. The synthetic fluorogenic compound used was obtained from Serva Fine Biochemicals, Inc., Garden City Park, L.I., N.Y. (Catalog #51474). This compound consists of a tripeptide (Gly-Gly-Arg) attached via an amide linkage to the fluorescent compound 7-amino-4-methyl coumarin (AMC); the amino group of glycine is blocked by carbobenzoxy chloride (Cbz). The structure of this compound (herein called Tripeptide-AMC) is shown below:

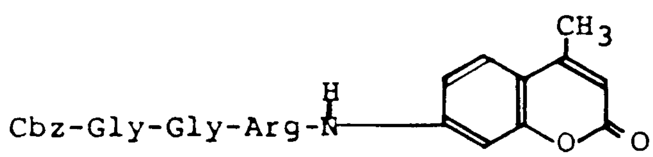

Tripeptide-AMC

The excitation and emission maxima of free AMC (345 nm and 445 nm, respectively) differ from those for AMC bound to the tripeptide (325 nm and 395 nm, respectively). This affords a means for distinguishing between the bound and free forms of the AMC molecule using a fluorometric assay. Excitation and emission wavelengths of 383 nm and 455 nm may be used for optimum differences for assay purposes; at these wavelengths, free AMC retains 20% of its maximal fluorescence but possesses a relative fluorescence 500-fold greater than an equimolar amount of bound AMC (Zimmerman, et al., 1978, Proc. Natl. Acad. Sci.,

U.S.A. 75(2):750-753).

In order to effect specific bonding of the Tripeptide-AMC to the oxidized carbohydrate moiety of the antibody prepared in Section 6.1, a hydrazine derivative was linked to the terminal glycine of the Tripeptide-AMC compound. The presence of the hydrazine group is advantageous since this results in reactivity for the oxidized carbohydrate moiety of the antibody molecule under very mild conditions while not affecting the antigen binding site. Aldehyde groups of the oxidized carbohydrate side chain of the antibody molecule then react with the hydrazine derivative to form a hydrazone.

In order to attach a hydrazine derivative (e.g., 4-fluorophenylhydrazine), the Tripeptide-AMC was first deblocked at the glycine amino terminus by removal of the Cbz group. This was accomplished by dissolving the Tripeptide-AMC in trifluoroacetic acid (Sigma, St. Louis, Mo.), and bubbling HBr gas (Matheson, East Rutherfurd, N.J.) through the solution for 45 minutes. The product, $H_2N$-Gly-Gly-Arg-NH-AMC, was precipitated by the addition of cold diethyl ether (Baker Chemical Co., Phillipsburg, N.J.), and dissolved in absolute ethanol (Publicker Industries Co., Linfield, Pa.). An equimolar amount of 4-fluorophenylhydrazine (Aldrich Chemical Co., Milwaukee, Wis.) in absolute ethanol was added with mixing. After incubation in the dark at room temperature for 2 hours, the reaction mixture was stored in the dark at 4°C. The resulting product (Phenylhydrazine-Tripeptide-AMC) has the structure:

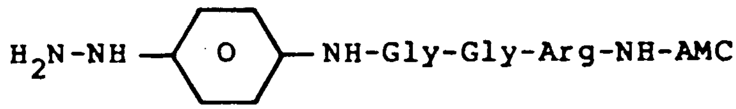

This compound was shown to be positive for fluorescence by exciting with ultraviolet light, and positive for the presence of a hydrazine group. The hydrazine linked to the tripeptide was detected by thin layer chromatography (TLC) using a spray of a 0.1% trinitrobenzene sulfonic acid aqueous solution for the colorimetric determination of a hydrazine (a pinkish or orange-brown color indicates the presence of hydrazine). The results of TLC demonstrated the presence of a hydrazine group at the migratory band of the Tripeptide-AMC.

The absorption and emission spectra for the Phenylhydrazine-Tripeptide-AMC compound as shown in FIG. 5 reveal a similarity to the Tripeptide-AMC spectra but a shift in excitation and emission maxima consistent with the covalent modification of the Tripeptide-AMC. The maxima for excitation and emission of the Phenylhydrazine-Tripeptide-AMC compound are 345 nm and 385 nm, respectively. The product was precipitated from solution with cold diethyl ether, washed, and dissolved in dimethylsulfoxide (Baker Chemical Co., Phillipsburg, N.J.),

### 6.3. COVALENT ATTACHMENT OF PHENYLHYDRAZIDE-TRIPEPTIDE-AMC TO THE OXIDIZED CARBOHYDRATE MOIETY OF THE ANTIBODY MOLECULE

The oxidized monoclonal antibody preparation, described in Section 6.1, supra, was adjusted to pH 5.1 by the addition of a small amount of 0.1M acetate buffer (pH 5.0). An estimated 10-fold excess of Phenylhydrazine-Tripeptide-AMC (prepared in Section 6.2) was added to the antibody solution, which was then incubated at 37°C in the dark, overnight (approximately 14 hours). The reaction mixture was then chromatographed on a Sephadex G-25 column (Pharmacia Fine Chemicals, Piscataway, N.J.) in order to remove any unreacted Phenylhydrazine-Tripeptide-AMC.

Spectrofluorometric analysis of the protein fractions confirmed the presence of the Phenylhydrazine-Tripeptide-AMC covalently attached to the antibody (now called Antibody-Phenylhydrazine-Tripeptide-AMC, or antibody conjugate). The excitation and emission maxima for the conjugate are 325 nm and 385 nm, respectively (FIG. 6). The large peak at 285 nm in the excitation spectrum of the conjugate may be explained by tryptophan absorption with residual fluorescence at 385 nm and may also be the result of resonance energy transfer from the amino acid tryptophan of the antibody molecule to AMC.

## 7. EXAMPLES: SERIES II

The purpose of this series of examples is to demonstrate that methods for preparing antibody conjugates of the invention do not adversely affect the antigen binding properties of antibodies in the way the carbodimide reaction affects such properties. To this end, the carbohydrate of a mouse monoclonal IgM, specific for the phosphorylcholine group, was oxidized and covalently attached to the 1,6-diaminohexyl

derivative of ethylene diamine di-(o-hydroxyphenylacetic acid) [EDDHA] to form 1,6-diaminohexyl-EDDHA. For comparative purposes, 1,6-diaminohexyl-EDDHA as well as unmodified EDDHA were attached to identical samples of the IgM monoclonal antibody using the carbodiimide reaction. Under these conditions, the 1,6-diaminohexyl-EDDHA would couple to available aspartic and glutamic acid residues, while the unmodified EDDHA would couple to available lysines.

The binding properties of these samples were compared with the native antibody in order to evaluate affinity and homogeneity.

### 7.1. OXIDATION OF MOUSE MONOCLONAL IgM

A mouse monoclonal IgM antibody specific for the ligand, phosphorylcholine, was oxidized at a concentration of 2 mg/mℓ in phosphate buffered saline (PBS, 0.01M phosphate, 0.15M sodium chloride), pH 7.0. The antibody-containing solution was cooled in a water-ice bath, and 56.8 µg of sodium metaperiodate was added (40 µℓ of a 1.42 mg/mℓ solution; final periodate concentration = 0.26mM). This reaction mixture was incubated for one hour, after which 2 µℓ of ethylene glycol was added. This was incubated an additional thirty minutes. The sample was then passed through a Sephadex G-25 column equilibrated with PBS and the protein fractions pooled.

### 7.2. ATTACHMENT OF LINKER TO EDDHA

EDDHA (1.5 g, 4.2 mmole) and triethylamine (1.2 mℓ, 8.4 mmole) were mixed with 40 mℓ of water. This heterogeneous solution was heated to 60 °C and stirred vigorously for 0.5 hour. The solution was dried in vacuo and then was dissolved in 400 mℓ of dry N,N-dimethylformamide. The solution was then cooled in an ice bath and isobutylchloroformate (0.56 mℓ, 4.2 mmole) was added. The reaction mixture was stirred with cooling for 0.5 hours. The resulting triethylamine hydrochloride precipitate was removed by filtration and the filtrate containing mixed carboxycarbonic anhydride of EDDHA was red in color.

1-amino-6-trifluoroacetamidohexane (0.8 g, 4.1 mmole) was added to the above carboxycarbonic anhydride of EDDHA. The homogeneous solution was stirred at 4 °C for 0.5 hour, then was lyophilized to yield an oily product. The oil was washed with acetone/ether (4:1) mixture to yield a crude yellow product. The solid 1-amino-6-trifluoroacetamidohexyl-EDDHA was collected and hydrolyzed with 7% $K_2CO_3$ and reprecipitated with HCl at pH 4 to yield pure 1,6-diaminohexyl-EDDHA (1.4 g). This compound gives a positive test of ninhydrin and thin layer chromatography shows only one spot. In the presence of basic solution of an equal molar quantity of $TbCl_3$, excitation at 295 nm yielded emission at 545 nm, due to formation of the characteristic energy transfer chelate complex between EDDHA and terbium ion.

### 7.3. PREPARATION OF IgM-LINKER-EDDHA CONJUGATES

The antibody, oxidized by the method of Section 7.1, was incubated with an approximately 270-fold molar excess of 1,6-diaminohexyl-EDDHA, prepared by the method of Section 7.2, for one hour at room temperature. This was followed by addition of solid sodium cyanoborohydride to a final concentration of 10mM, and further incubation of 4 hours at room temperature. The mixture was then dialyzed at 4 °C versus several changes of PBS, and concentrated by ultrafiltration.

### 7.4. CARBODIIMIDE ATTACHMENT OF LINKER-EDDHA TO IgM

To 263 µℓ IgM antibody (1.9 mg/mℓ) was added 10 mg of 1-ethyl-3-(3-dimethylaminopropyl) carbodiimide (1 mℓ of 10 mg/mℓ solution, pH 5.0) and PBS (pH 5.0) to make up to 2.5 mℓ. The mixture was incubated for two hours at room temperature. Then 275 µℓ of 0.1M 1,6-diaminohexyl-EDDHA in 2.5 mℓ water (pH 5.5) was added and the solution incubated two hours at room temperature. Ten µl of 1M ethanolamine was then added and incubated for one hour at room temperature. This was then dialysed overnight against PBS (pH 7.0).

### 7.5. CARBODIIMIDE ATTACHMENT OF EDDHA TO IgM

To 263 µℓ IgM antibody (1.9 mg/mℓ) was added 10 mg of 1-ethyl-3-(3-dimethylaminopropyl) carbodiimide (1 mℓ of 10 mg/mℓ solution, pH 5.0) and PBS (pR 5.0) to make up to 2.5 mℓ. The mixture was incubated for two hours at room temperature. To this was added 2.75 mℓ of 0.01M EDDHA (pH 5.5) and the solution was incubated for two hours at room temperature. Ten µℓ 1M ethanolamine was added and the

mixture incubated for one hour at room temperature. This was then dialysed overnight against PBS (pH 7.0).

7.6. EFFECTS OF CARBOHYDRATE-MEDIATED ATTACHMENT OF ANTIBODIES

The affinities of unmodified mouse monoclonal antibody and antibody conjugates prepared according to Sections 7.3, 7.4 and 7.5, all specific for the phosphorylcholine group, were measured by fluozescence quenching using a Perkin-Elmin Model 512 Double Beam Fluorescence Spectrometer (Perkin-Elmer Corporation, Norwalk, Connecticut). The excitation and emission wavelengths were 295 and 345 nm, respectively, and the temperature was maintained at 25°C with a Lauda K-2/R circulating water both (Brinkmann Instruments).

Antibody concentrations were calculated using an

$$\varepsilon^{280}_{1\%} = 13.5$$

and ranged from 7.5 x $10^{-8}$ to 9.7 x $10^{-8}$ M. All binding sites were assumed to be active and the sample volumes were 3.0 mℓ. The stock concentrations of the ligand, N-(2,4-dinitrophenyl)-p-aminophenylphosphorylcholine (DPPC), was 1.17 x $10^{-4}$ M. The titrations were done in Phosphate-buffered saline at pH 7.4. The ligand was added continuously, with stirring, at a rate of 9.01 μℓ/min using a motor-driven syringe, and the fluorescence intensity was monitored by a CBM 2001 series computer equipped with a 2040 dual drive floppy disk and a 2023 printer (Commodore Business Machines), interfaced with a Preset I, analog/digital converter (Connecticut Microcomputers). To correct for attenuation and sample dilution, a non-specific human Waldenström's macroglobulin or anti-lactose murine hybridoma antibodies [Mandal and Karush, 1981, J. Immunol. 127:1240] were titrated. A total of 180 μℓ of ligand solution as added per titration. Binding parameters were calculated using Sips distribution function [Sips, 1948, J. Chem. Phys. 16:490] in a reiterative manner, in which the maximum quench was allowed to vary until the heterogeneity index was within 0.002 of unity.

The calculation of the $Q_{max}$ values involves a significant uncertainty, probably not less than 10%, due to the reiterative procedure used to generate them. This uncertainty arises from the fact that any discrepancy between the actual concentration of active sites and that calculated from the optical density is compensated for in the derived value of $Q_{max}$ when the criterion of a heterogeneity index of unity is employed. This compensation results from the dependence of the concentration of bound ligand on the ratio of antibody concentration to the value of $Q_{max}$. Because of this dependence the accuracy of the derived values of the association constants is not limited by the concentration of active sites.

The Sips plots presenting data for unmodified antibody carbodiimide conjugates and antibody conjugates of the invention are shown in FIG. 7. The binding measurements clearly demonstrate the retention of specificity, affinity, and homogeneity for the sample modified via the carbohydrate attachment methods of the invention

(Δ—Δ),

when compared to the unmodified antibody

(●—●).

The association constant for the binding of the phosphorylcholine derivative was measured to be 0.1 x $10^5$ $M^{-1}$ for the unmodified antibody and 1.1 x $10^6$ $M^{-1}$ for the carbohydrate-attached antibody conjugate. In contrast to this, an antibody preparation modified by the carbodiimide reaction

(□—□)

has substantially reduced binding, certainly well below the calculated values of 1-2 x $10^5$. The assumption of a heterogeneity index of unity in the Sips analysis is valid for the data reduction only if the sample is

homogeneous (monoclonal). A check on the actual homogeneity (monoclonal nature) of the sample is the correlation coefficient or fit if the experimental data points with the calculated line in the Sips plot. Inspection of the plots of FIG. 7 clearly show good agreement for the unmodified antibody and carbohydrate-attached antibody and very poor agreement for those with carbodiimide-attachments. This is most likely due to the lack of selectivity of the carbodiimide attachment method. Lysines, glutamic and aspartic acids occur in all parts of antibody molecules, including the antigen binding regions. As a result, at least some of the antibodies are modified at or near the binding sites with consequent effects or interaction with antigen. The sites of attachment to carbohydrate, however, are specific and distal from the binding site, and provide little, if any, change in binding properties observed in these experiments. While obtained for covalently modified antibodies in solution, it is believed that the conclusions of this study may be extended to immobilized antibodies as well (identical experiments with immobilized antibodies cannot be done for technical reasons). The random nature of commonly used attachment chemistries (cyanogen bromide activated gels, glutaraldehyde beads, N-hydroxysuccinimide ester gels, etc.) ought to yield matrices with reduced binding capacity when compared with site-specific attachments of the invention.

## 8. EXAMPLES: SERIES III

The following examples illustrate a method for the specific attachment to an antibody molecule of a peptide linked to a compound of interest (compound) via an amide or ester bond. The resulting antibody conjugate retains the ability to fix complement as revealed by a hemolytic complement fixation assay. Furthermore, the specific release of the compound at the antigenic cell surface via enzymatic cleavage by the complement system is demonstrated by a non-hemolytic assay.

In the following examples the compound is fluorogenic. Thus, the complement mediated release of the fluorescent compound may be detected by an assay capable of differentiating between the bound and free forms of the fluorescent molecule.

The materials and procedures of Section 6.1 were used as described to oxidize the carbohydrate moieties of monoclonal antibodies (No. 171).

In the presence of sheep red blood cells and serum complement, these monoclonal antibodies (No. 171) activate the complement enzyme cascade (a result of antigen-antibody binding). Complement fixation causes lysis of the sheep red blood cells which results in the release of hemoglobin. The released hemoglobin may be detected spectrophotometrically, thus providing an assay for complement fixation.

The tripeptide-AMC was prepared as described in Section 6.2. The properties of the fluorogenic compound (AMC) are such that the bound and free states of the fluorogenic compound are spectrofluorometrically distinguishable. This provides a definitive assay for measuring the complement fixation ability of the antibody conjugate. More importantly, it provides a means for quantitating the subsequent complement-mediated release of the compound.

The specific covalent attachment of Phenylhydrazine-tripeptide-AMC to the oxidized carbohydrate moieties of the antibodies was performed as described in Section 6.3.

### 8.1 COMPLEMENT FIXATION ASSAYS

Two types of complement fixation assays were utilized, hemolytic and fluorometric. These assays determined whether the Antibody-Phenylhydrazine-Tripeptide-AMC conjugate retained complement fixation ability, and whether AMC was cleaved by complement.

#### 8.1.1 PREPARATION OF HUMAN COMPLEMENT

A 10 mℓ sample of freshly drawn human whole blood was clotted on ice for 17 hours. The clot was removed by centrifugation, and the resulting human serum was frozen in 0.5 mℓ aliquots. Human complement was shown to be active in these samples by the hemolytic assay described in Section 8.1.2.

#### 8.1.2. HEMOLYTIC ASSAY FOR COMPLEMENT FIXATION

A 200 μℓ aliquot of a suspension of sheep red blood cells (Gibco Diagnostics, Madison, Wis.) at an approximate concentration of 2 x $10^8$ cells/mℓ were mixed with 20 μℓ of the antibody conjugate mixture prepared in Section 6.3 (approximately 2 μg of protein). After 15 minutes of mixing and incubating at 37° C, 100 μℓ of the human serum complement (prepared in Section 8.1.1) was added to the mixture. After 30 min to 1 hour of incubation at 37° C, the mixture was centrifuged to pellet the cells. The extent of complement-mediated cell lysis was determined by spectrophotometrically measuring hemoglobin released into the supernatant (412 nm).

The results of this assay demonstrated complete hemolysis and essentially 100% binding of antibody to cell surface. For example, addition of distilled water to a pellet formed by centrifuging 200 µℓ of the sheep red blood cell suspension completely lyses the cells, and releases hemoglobin. A 1:20 dilution of the supernatant of sheep red blood cells which were completely lysed in distilled water had an $O.D._{412}$ of 0.646. An identical dilution of sheep red blood cells which were lysed by the addition of conjugate and complement had an $O.D._{412}$ of 0.672. Thus the conjugate retained the ability to bind antigen and to fix complement.

8.1.3 NON-HEMOLYTIC ASSAY FOR COMPLEMENT MEDIATED RELEASE OF AMC

Conditions for the non-hemolytic assay were identical to those above except that glutaraldehyde-fixed sheep red blood cells (Sigma, St. Louis, Mo.) were used in place of normal sheep red blood cells. Glutaraldehyde fixed cells do not lyse in the presence of antibody and complement and, therefore, no hemoglobin is released. Instead, a fluorometric assay is used to demonstrate the release of the AMC. A non-hemolytic system is necessary for use in the fluorometric assay, because the presence of hemoglobin interferes with fluorescence measurements in this system. Prior to use in the assay, these fixed red blood cells were shown to bind both the unmodified antibody and the Atbody-Phenylhydrazine-Tripeptide-AMC which was prepared in Section 6.3.

The non-hemolytic assay was used to show the specific complement-mediated release of the AMC from the antibody conjugate. Similarly to the hemolytic assay, 200 µℓ of the glutaraldehyde-fixed sheep red blood cells, at an approximate concentration of 2 x $10^8$ cells/mℓ, was incubated with the Antibody-Phenylhydrazide-Tripeptide-AMC conjugate at 37° C for 15 minutes.

After centrifuging and resuspension in buffer, 50 µℓ of the human complement preparation (Section 8.1.1) was added, and the fluorescense at 460 nm monitored, with excitation at 380 nm (Caporale, et al., 1981, J. Immunol. 128 1963-65.) as a function of time. As controls, the conjugate was incubated with sheep red blood cells alone; in the presence of rat red blood cells and human complement (the monoclonal antibody used does not bind to rat red blood cells); and in the absence of both sheep red blood cells and complement (the monoclonal antibody used does not bind to rat red blood cells). FIG. 8 shows the results of these experiments. A comparision of curve (a) which represents the conjugate incubated with glutaraldehyde-fixed sheep red blood cells and human complement to the control curves labeled (b), (c) and (d) clearly demonstrates the release of free AMC in the sample containing the specific antibody target and human complement. Thus, curve (b) which represents the conjugate incubated with glutaraldehyde-fixed rat red blood cells and human complement, curve (c) which represents the conjugate incubated with glutaraldehyde-fixed sheep red blood cells, and curve (d) which represents the conjugate alone demonstrate no release of AMC.

**Claims**

1. A soluble antibody conjugate for a medical use comprising: a water-soluble therapeutic or diagnostic compound containing an amine group selected from the group consisting of secondary amine, hydrazine, hydrazide, hydroxylamine, phenylhydrazine and semicarbazide, attached through a covalent bond to an aldehyde group of an oxidized carbohydrate moiety of an antibody or antibody fragment said covalent bond between said amine group and said aldehyde group being an enamine, hydrazone, oxime, phenylhydrazone, semicarbazone or a reduced form thereof, and said soluble antibody conjugate having (i) substantially the same immunoreactivity and immunospecificity as the unconjugated antibody or antibody fragment, and (ii) aqueous solubility, such that the conjugate is suitable for in vivo administration.

2. The antibody conjugate according to claim 1, wherein the antibody fragment is selected from the group consisting of Fab fragments, $(Fab')_2$ fragments and half antibody molecules.

3. The antibody conjugate according to claim 2, wherein the antibody is a monoclonal antibody or a monoclonal antibody fragment.

4. The antibody conjugate according to claims 1 to 3, wherein the compound is a protein or an enzyme.

**5.** The antibody conjugate according to claims 1 to 3, wherein the compound is a hormone.

**6.** The antibody conjugate according to claims 1 to 3, wherein the compound is a catalyst.

**7.** The antibody conjugate according to claims 1 to 3, wherein the compound is a fluorescent or potentially fluorescent compound or a chemiluminescent compound.

**8.** The antibody conjugate according to claims 1 to 3, wherein the compound is a chelating agent.

**9.** A soluble antibody conjugate for a medical use, comprising: a water-soluble therapeutic or diagnostic compound which comprises a water-soluble linker attached to a second compound containing an amine group selected from the group consisting of primary amine, secondary amine, hydrazine, hydrazide, hydroxylamine; phenylhydrazine and semicarbazide attached through a covalent bond to an aldehyde group of an oxidized carbohydrate moiety of an antibody or antibody fragment said covalent bond between said amine group and said aldehyde group being an imine, enamine, hydrazone, oxime, phenylhydrazone, semicarbazone or a reduced form thereof, and said soluble antibody conjugate having (i) substantially the same immunoreactivity and immunospecificity as the conjugated antibody or antibody fragment, and (ii) aqueous solubility such that the conjugate is suitable for in vivo administration.

**10.** The antibody conjugate according to claim 9, wherein the water-soluble linker is a heterobifunctional linker.

**11.** The antibody conjugate according to claim 9, wherein the water-soluble linker is mercaptoethanolamine or the tripeptide gly-gly-arg.

**12.** An antibody conjugate according to claims 9 to 11, wherein the second compound is an enzyme.

**13.** An antibody conjugate according to claims 9 to 11, wherein the second compound is a hormone.

**14.** An antibody conjugate according to claims 9 to 11, wherein the second compound is a catalyst.

**15.** An antibody according to claims 9 to 11, wherein the second compound is a fluorescent or potentially fluorescent compound or a chemiluminescent or potentially chemiluminescent compound.

**16.** An antibody conjugate according to claim 9 wherein the second compound is a chelating agent attached to a metal.

**17.** An antibody conjugate according to claims 1 to 19, wherein the antibody conjugate is stabilized by exposure to an effective amount of a reducing agent.

**18.** An antibody conjugate according to claims 1 or 9 wherein the water-soluble compound is a linker selected from the group consisting of a peptide linker, an amino acid linker and a linker of the general formula

$W-(CH_2)_n-Q$

Wherein W is either

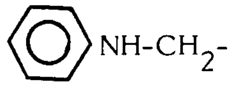 or

$-CH_2-$ ; Q is an amino acid, peptide, chelator or chelator derivative ; and n is an integer from 0 to 20 ; and the linker being attached via a covalent bond or coordinate bond to a second compound.

**19.** An antibody conjugate of claim 18 wherein the linker is a peptide which is a substrate for an activated

complement component.

**20.** An antibody conjugate of claim 18 wherein the antibody is capable of activating serum complement when bound to antigen and the covalent bond between the linker and compound is not susceptible to cleavage by activated complement, the resulting antibody conjugate retaining the ability to activate complement.

**21.** An antibody conjugate of claim 18, wherein the antibody is capable of activating serum complement when bound to antigen and the covalent bond between the linker and the compound is susceptible to cleavage by activated complement.

**22.** An antibody conjugate of claim 18, wherein the antibody is not capable of activating serum complement when bound to antigen and the covalent bond between the linker and the compound is susceptible to cleavage by activated complement.

**23.** An antibody conjugate of claim 18, wherein the antibody is not capable of activating serum complement when bound to antigen and the covalent bond between the linker and the compound is not susceptible to cleavage by activated complement.

**24.** An antibody conjugate of claim 18, wherein the antibody is not capable of activating serum complement when bound to antigen and the covalent bond between the linker and the compound is susceptible to cleavage by serum proteases.

**25.** An antibody conjugate of claim 18, wherein the covalent bond between the linker and the compound is susceptible to cleavage by activated complement.

**26.** An antibody conjugate of claim 18, wherein the covalent bond between the linker and the compound is not susceptible to cleavage by activated complement.

**27.** An antibody conjugate of claim 18, wherein the covalent bond between the linker and the compound is not susceptible to cleavage by serum proteases.

**28.** An antibody conjugate according to claim 18 for delivery of the compound at an antigenic site, wherein the antibody or antibody fragment is directed against said antigenic site.

**29.** An antibody conjugate according to claim 28, wherein the antigenic site is a bacterial antigen and the compound is an antibacterial agent.

**30.** An antibody conjugate according to claim 28, wherein, the antigenic site is a viral antigen and the compound is an antiviral agent.

**31.** An antibody conjugate according to claim 28, wherein the antigenic site is a tumor antigen and the compound is an antitumor agent.

**32.** An antibody conjugate according to claim 31, wherein the antitumor agent is selected among fluorouracil, bleomycin, adriamycin, cerubidine, valban and alkeran.

**33.** An antibody conjugate according to claim 28, wherein the antigenic site is a fungal antigen and the compound is an antifungal agent.

**34.** An antibody conjugate according to claim 28, wherein the antigenic site is a parasite antigen and the compound is antiparasitic agent.

**35.** An antibody conjugate according to claim 28, wherein the antigenic site is a mycoplasmal antigen and the compound is an antimycoplasmal agent.

**36.** An antibody conjugate according to claim 28, wherein the antigenic site is a differentiation or histocompatability antigen and the compound is a cytotoxic agent.

37. An antibody conjugate according to claim 28, wherein the compound is a radiopharmaceutical or a metal.

38. An antibody conjugate according to claim 37, wherein the metal is platinum.

39. An antibody conjugate according to claim 28, wherein the compound is a toxin or toxin fragment.

40. An antibody conjugate according to claim 28, wherein the compound is neurotransmitter or hormone.

41. An antibody conjugate according to claim 28, wherein the compound is an enzyme or a DNA sequence.

**Revendications**

1. Conjugué d'anticorps soluble pour application médicale comprenant: un composé thérapeutique ou diagnostique soluble contenant un groupe amine choisi dans le groupe constitué par amine secondaire, hydrazine, hydrazide, hydroxylamine, phénylhydrazine et semicarbazide, attaché par une liaison covalente à un groupe aldéhyde d'une partie hydrate de carbone oxydée d'un anticorps ou fragment d'anticorps, ladite liaison covalente entre ledit groupe amine et ledit groupe aldéhyde étant une énamine, une hydrazone, un oxime, une phénylhydrazone, une semicarbazone ou une de leurs formes réduites, et ledit conjugué d'anticorps soluble ayant (i) sensiblement la même immunoréactivité et la même immunospécificité que l'anticorps ou fragment d'anticorps non conjugué, et (ii) une solubilité dans l'eau, si bien que le conjugué convient pour l'administration in vivo.

2. Conjugué d'anticorps selon la revendication 1, dans lequel le fragment d'anticorps est choisi dans le groupe constitué par les fragments Fab, les fragments $(Fab')_2$ et les molécules semi-anticorps.

3. Conjugué d'anticorps selon la revendication 2, dans lequel l'anticorps est un anticorps monoclonal ou fragment d'anticorps monoclonal.

4. Conjugué d'anticorps selon les revendications 1 à 3, dans lequel le composé est une protéine ou une enzyme.

5. Conjugué d'anticorps selon les revendications 1 à 3, dans lequel le composé est une hormone.

6. Conjugué d'anticorps selon les revendications 1 à 3, dans lequel le composé est un catalyseur.

7. Conjugué d'anticorps selon les revendications 1 à 3, dans lequel le composé est un composé fluorescent ou potentiellement fluorescent ou un composé chimioluminescent.

8. Conjugué d'anticorps selon les revendications 1 à 3, dans lequel le composé est un agent chélant.

9. Conjugué d'anticorps soluble pour application médicale, comprenant: un composé thérapeutique ou diagnostique soluble dans l'eau qui comprend un liant soluble dans l'eau attaché à un second composé contenant un groupe amine choisi dans le groupe constitué par amine primaire, amine secondaire, hydrazine, hydrazide, hydroxylamine; phénylhydrazine et semicarbazide attaché par une liaison covalente à un groupe aldéhyde d'une partie hydrate de carbone oxydée d'un anticorps ou fragment d'anticorps, ladite liaison covalente entre ledit groupe amine et ledit groupe aldéhyde étant une imine, une énamine, une hydrazone, un oxime, une phénylhydrazone, une semicarbazone ou une de leurs formes réduites, et ledit conjugué d'anticorps soluble ayant (i) sensiblement la même immunoréactivité et la même immunospécificité que l'anticorps ou fragment d'anticorps conjugué, et (ii) une solubilité dans l'eau telle que le conjugué convient pour l'administration in vivo.

10. Conjugué d'anticorps selon la revendication 9, dans lequel le liant soluble dans l'eau est un liant hétérobifonctionnel.

11. Conjugué d'anticorps selon la revendication 9, dans lequel le liant soluble dans l'eau est la mercaptoéthanolamine ou le tripeptide Gly-Gly-Arg.

**12.** Conjugué d'anticorps selon les revendications 9 à 11, dans lequel le second composé est une enzyme.

**13.** Conjugué d'anticorps selon les revendications 9 à 11, dans lequel le second composé est une hormone.

**14.** Conjugué d'anticorps selon les revendications 9 à 11, dans lequel le second composé est un catalyseur.

**15.** Anticorps selon les revendications 9 à 11, dans lequel le second composé est un composé fluorescent ou potentiellement fluorescent ou un composé chimioluminescent ou potentiellement chimioluminescent.

**16.** Conjugué d'anticorps selon la revendication 9 dans lequel le second composé est un agent chélant attaché à un métal.

**17.** Conjugué d'anticorps selon les revendications 1 à 19 dans lequel le conjugué d'anticorps est stabilisé par exposition à une quantité efficace d'un agent réducteur.

**18.** Conjugué d'anticorps selon les revendications 1 ou 9 dans lequel le composé soluble dans l'eau est un liant choisi dans le groupe constitué par un liant peptidique, un liant acide aminé et un liant de formule générale

$W-(CH_2)_n-Q$

dans laquelle W représente

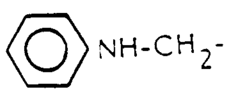

ou $-CH_2-$ ; Q est un acide aminé, un peptide, un chélateur ou un dérivé de chélateur; et n est un nombre entier valant de 0 à 20; et le liant étant attaché via une liaison covalente ou une liaison de coordination à un second composé.

**19.** Conjugué d'anticorps de la revendication 18 dans lequel le liant est un peptide qui est un substrat pour un composant complément activé.

**20.** Conjugué d'anticorps de la revendication 18 dans lequel l'anticorps est capable d'activer le complément de sérum lorsqu'il est lié à l'antigène et la liaison de covalence entre le liant et le composé n'est pas susceptible d'être coupée par le complément activé, le conjugué d'anticorps résultant conservant l'aptitude à activer le complément.

**21.** Conjugué d'anticorps de la revendication 18, dans lequel l'anticorps est capable d'activer le complément de sérum lorsqu'il est lié à l'antigène et la liaison de covalence entre le liant et le composé est susceptible d'être coupée par le complément activé.

**22.** Conjugué d'anticorps de la revendication 18, dans lequel l'anticorps n'est pas capable d'activer le complément de sérum lorsqu'il est lié à l'antigène et la liaison de covalence entre le liant et le composé est susceptible d'être coupée par le complément activé.

**23.** Conjugué d'anticorps de la revendication 18, dans lequel l'anticorps n'est pas capable d'activer le complément de sérum lorsqu'il est lié à l'antigène et la liaison de covalence entre le liant et le composé n'est pas susceptible d'être coupée par le complément activé.

**24.** Conjugué d'anticorps de la revendication 18, dans lequel l'anticorps n'est pas capable d'activer le complément de sérum lorsqu'il est lié à l'antigène et la liaison de covalence entre le liant et le composé est susceptible d'être coupée par des protéases sériques.

**25.** Conjugué d'anticorps de la revendication 18, dans lequel la liaison de covalence entre le liant et le composé est susceptible d'être coupée par le complément activé.

**26.** Conjugué d'anticorps de la revendication 18, dans lequel la liaison de covalence entre le liant et le composé n'est pas susceptible d'être coupée par le complément activé.

**27.** Conjugué d'anticorps de la revendication 18, dans lequel la liaison de covalence entre le liant et le composé n'est pas susceptible d'être coupée par des protéases sériques.

**28.** conjugué d'anticorps selon la revendication 18 pour délivrance du composé en un site antigénique, où l'anticorps ou fragment d'anticorps est dirigé contre ledit site antigénique.

**29.** Conjugué d'anticorps selon la revendication 28, dans lequel le site antigénique est un antigène bactérien et le composé est un agent antibactérien.

**30.** Conjugué d'anticorps selon la revendication 28, dans lequel le site antigénique est un antigène viral et le composé est un agent antiviral.

**31.** Conjugué d'anticorps selon la revendication 28, dans lequel le site antigénique est un antigène tumoral et le composé est un agent antitumoral.

**32.** Conjugué d'anticorps selon la revendication 31, dans lequel l'agent antitumoral est choisi parmi le fluorouracile, la bléomycine, l'adriamycine, la cérubidine, le valban et l'alkéran.

**33.** Conjugué d'anticorps selon la revendication 28, dans lequel le site antigénique est un antigène fongique et le composé est un agent antifongique.

**34.** Conjugué d'anticorps selon la revendication 28, dans lequel le site antigénique est un antigène parasite et le composé est un agent antiparasitaire.

**35.** Conjugué d'anticorps selon la revendication 28, dans lequel le site antigénique est un antigène mycoplasmique et le composé est un agent antimycoplasmique.

**36.** Conjugué d'anticorps selon la revendication 28, dans lequel le site antigénique est un antigène de différentiation ou d'histocompatibilité et le composé est un agent cytotoxique.

**37.** Conjugué d'anticorps selon la revendication 28, dans lequel le composé est un corps radiopharmaceutique ou un métal.

**38.** Conjugué d'anticorps selon la revendication 37 dans lequel le métal est le platine.

**39.** conjugué d'anticorps selon la revendication 28, dans lequel le composé est une toxine ou un fragment de toxine.

**40.** Conjugué d'anticorps selon la revendication 28, dasn lequel le composé est un neurotransmetteur ou une hormone.

**41.** Conjugué d'anticorps selon la revendication 28, dans lequel le composé est une enzyme ou une séquence d'ADN.

## Patentansprüche

**1.** Eine lösliche Antikörperverbindung für medizinische Zwecke, die folgendes umfaßt:
Eine wasserlösliche, therapeutische oder diagnostische Verbindung, bestehend aus einer Amingruppe, die aus der Gruppe selektiert wird, welche aus Sekundäramin, Hydrazin, Hydrazid, Hydroxylamin, Phenylhydrazin und Semikarbazid besteht, verbunden durch eine Kovalenzbindung mit einer Aldehydgruppe eines oxidierten Kohlenhydratanteils eines Antikörpers oder Antikörperfragments, wobei die besagte Kovalenzbindung zwischen besagter Amingruppe und besagter Aldehydgruppe ein Enamin,

Hydrazon, Oxim, Phenylhydrazon, Semikarbazon oder eine reduzierte Form derselben ist, und wobei besagte lösliche Antikörperverbindung (i) im wesentlichen dieselbe Immunisierungsreaktivität und Immunisierungsspezifizität, wie der nichtgekoppelte Antikörper oder das nicht-gekoppelte Antikörperfragment besitzt, und (ii) eine Wasserlöslichkeit aufweist, damit die Verbindung für die in vivo Verabreichung geeignet ist.

**2.** Antikörperverbindung nach Anspruch 1, worin das Antikörperfragment aus der Gruppe selektiert wird, die aus Fab-Fragmenten, $(Fab')_2$-Fragmenten und Halbantikörpermolekülen besteht.

**3.** Antikörperverbindung nach Anspruch 2, worin der Antikörper ein monoklonaler Antikörper oder ein monoklonales Antikörperfragment ist.

**4.** Antikörperverbindung nach den Ansprüchen 1 bis 3, worin die Verbindung ein Protein oder ein Enzym ist.

**5.** Antikörperverbindung nach den Ansprüchen 1 bis 3, worin die Verbindung ein Hormon ist.

**6.** Antikörperverbindung nach den Ansprüchen 1 bis 3, worin die Verbindung ein Katalysator ist.

**7.** Antikörperverbindung nach den Ansprüchen 1 bis 3, worin die Verbindung eine fluoreszente oder potentiell fluoreszente Verbindung oder eine chemilumineszente Verbindung ist.

**8.** Antikörperverbindung nach den Ansprüchen 1 bis 3, worin die Verbindung ein Chelatmittel ist.

**9.** Eine lösliche Antikörperverbindung für medizinische Zwecke, bestehend aus einem wasserlöslichen, therapeutischen oder diagnostischen Mittel, das ein wasserlösliches Bindeglied umfaßt, welches an eine zweite Verbindung, die eine Amingruppe enthält, angegliedert ist, wobei die Aminogruppe selektiert ist aus einer Gruppe, bestehend aus Primäramin, Sekundäramin, Hydrazin, Hydrazid, Hydroxylamin; wobei Phenylhydrazin und Semikarbazid mittels einer Kovalenzbindung einer Aldehydgruppe eines oxidierten Kohlenhydratanteils eines Antikörpers oder Antikörperfragments angegliedert sind, und wobei besagte Kovalenzbindung zwischen besagter Amingruppe und besagter Aldehydgruppe ein Amin, Enamin, Hydrazon, Oxim, Phenylhydrazon, Semikarbazon oder eine reduzierte Form derselben ist, und wobei besagte lösliche Antikörperverbindung (i) im wesentlichen dieselbe Immunisierungsreaktivität und Immunisierungsspezifizität, wie der gekoppelte Antikörper oder das gekoppelte Antikörperfragment besitzt, und (ii) die Wasserlöslichkeit so ist, daß die Verbindung für in vivo Verabreichung geeignet ist.

**10.** Antikörperverbindung nach Anspruch 9, worin das wasserlösliche Bindeglied ein hetero-bifunktionales Bindeglied ist.

**11.** Antikörperverbindung nach Anspruch 9, worin das wasserlösliche Bindeglied Mercaptoäthanolamin oder das Tripeptid gly-gly-arg ist.

**12.** Antikörperverbindung nach den Ansprüchen 9 bis 11, worin die zweite Verbindung ein Enzym ist.

**13.** Antikörperverbindung nach den Ansprüchen 9 bis 11, worin die zweite Verbindung ein Hormon ist.

**14.** Antikörperverbindung nach den Ansprüchen 9 bis 11, worin die zweite Verbindung ein Katalysator ist.

**15.** Antikörperverbindung nach den Ansprüchen 9 bis 11, worin die zweite Verbindung eine fluoreszente oder potentiell fluoreszente Verbindung oder eine chemilumineszente oder potentiell chemilumineszente Verbindung ist.

**16.** Antikörperverbindung nach Anspruch 9, worin die zweite Verbindung ein Chelatmittel ist, das einem Metall angegliedert ist.

**17.** Antikörperverbindung nach den Ansprüchen 1 bis 19, worin die Antikörperverbindung durch Aussetzung gegenüber einer wirksamen Menge eines reduzierenden Mittels stabilisiert wird.

**18.** Antikörperverbindung nach den Ansprüchen 1 oder 9, worin die wasserlösliche Verbindung ein Bindeglied ist, selektiert aus einer Gruppe, die aus einem Peptidbindeglied, einem Aminosäurebindeglied und einem Bindeglied der allgemeinen Formel

$W\text{-}(CH_2)_n\text{-}Q$

besteht, wobei W entweder

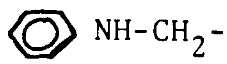

oder $-CH_2-$ ist; Q eine Aminosäure, ein Peptid, ein Chelat oder Chelatderivat ist; und n ein Ganzes zwischen 0 und 20 ist; und das Bindeglied durch eine Kovalenzbindung oder Koordinatbindung einer zweiten Verbindung angegliedert ist.

**19.** Antikörperverbindung nach Anspruch 18, worin das Bindeglied ein Peptid ist, das ein Substrat für eine aktivierte Komplementkomponente ist.

**20.** Antikörperverbindung nach Anspruch 18, worin der Antikörper in der Lage ist, Serumkomplement zu aktivieren, wenn dieses mit Antigen verbunden ist und die Kovalenzbindung zwischen Bindeglied und Verbindung durch das aktivierte Komplement nicht anfällig gegenüber Spaltung ist, wobei die sich ergebende Antikörperverbindung die Fähigkeit zur Aktivierung des Komplements behält.

**21.** Antikörperverbindung nach Anspruch 18, worin der Antikörper zur Aktivierung von Serumkomplement fähig ist, wenn er mit Antigen verbunden ist und die Kovalenzbindung zwischen dem Bindeglied und der Verbindung durch das aktivierte Komplement gegenüber Spaltung anfällig ist.

**22.** Antikörperverbindung nach Anspruch 18, worin der Antikörper zur Aktivierung von Serumkomplement nicht fähig ist, wenn er mit Antigen verbunden ist und die Kovalenzbindung zwischen dem Bindeglied und der Verbindung durch das aktivierte Komplement gegenüber Spaltung anfällig ist.

**23.** Antikörperverbindung nach Anspruch 18, worin der Antikörper zur Aktivierung von Serumkomplement nicht fähig ist, wenn er mit Antigen verbunden ist und die Kovalenzbindung zwischen dem Bindeglied und der Verbindung durch das aktivierte Komplement gegenüber Spaltung nicht anfällig ist.

**24.** Antikörperverbindung nach Anspruch 18, worin der Antikörper zur Aktivierung von Serumkomplement nicht fähig ist, wenn er mit Antigen verbunden ist und die Kovalenzbindung zwischen dem Bindeglied und der Verbindung durch Serumprotease gegenüber Spaltung anfällig ist.

**25.** Antikörperverbindung nach Anspruch 18, worin die Kovalenzbindung zwischen dem Bindeglied und der Verbindung durch aktiviertes Komplement gegenüber Spaltung anfällig ist.

**26.** Antikörperverbindung nach Anspruch 18, worin die Kovalenzbindung zwischen dem Bindeglied und der Verbindung durch aktiviertes Komplement gegenüber Spaltung nicht anfällig ist.

**27.** Antikörperverbindung nach Anspruch 18, worin die Kovalenzbindung zwischen dem Bindeglied und der Verbindung durch Serumprotease gegenüber Spaltung nicht anfällig ist.

**28.** Antikörperverbindung nach Anspruch 18 zwecks Lieferung der Verbindung an eine antigene Stelle, worin der Antikörper oder das Antikörperfragment gegen die besagte antigene Stelle gerichtet ist.

**29.** Antikörperverbindung nach Anspruch 28, worin die antigene Stelle ein bakterielles Antigen ist und die Verbindung ein antibakterielles Mittel ist.

**30.** Antikörperverbindung nach Anspruch 28, worin die antigene Stelle ein Virenantigen und die Verbindung ein Antivirenmittel ist.

**31.** Antikörperverbindung nach Anspruch 28, worin die antigene Stelle ein Tumorantigen und die Verbindung ein Antitumormittel ist.

**32.** Antikörperverbindung nach Anspruch 31, worin das Antitumormittel aus Fluorouracil, Bleomycin, Adriamycin, Cerubidin, Valban und Alkeran selektiert wird.

**33.** Antikörperverbindung nach Anspruch 28, worin die antigene Stelle ein pilzartiges Antigen und die Verbindung ein Antipilzmittel ist.

**34.** Antikörperverbindung nach Anspruch 28, worin die antigene Stelle ein Parasitantigen und die Verbindung ein Antiparasitmittel ist.

**35.** Antikörperverbindung nach Anspruch 28, worin die antigene Stelle ein mycoplasmisches Antigen und die Verbindung ein antimycoplasmisches Mittel ist.

**36.** Antikörperverbindung nach Anspruch 28, worin die antigene Stelle ein Differenzierungs- oder Histokompatibilitätsantigen und die Verbindung ein zytotoxisches Mittel ist.

**37.** Antikörperverbindung nach Anspruch 28, worin die Verbindung röntgenpharmazeutischer Natur oder ein Metall ist.

**38.** Antikörperverbindung nach Anspruch 37, worin das Metall Platin ist.

**39.** Antikörperverbindung nach Anspruch 28, worin die Verbindung ein Toxin oder Toxinfragment ist.

**40.** Antikörperverbindung nach Anspruch 28, worin die Verbindung ein Neurotransmitter oder ein Hormon ist.

**41.** Antikörperverbindung nach Anspruch 28, worin die Verbindung ein Enzym oder eine DNA-Sequenz ist.

FIG. 1a

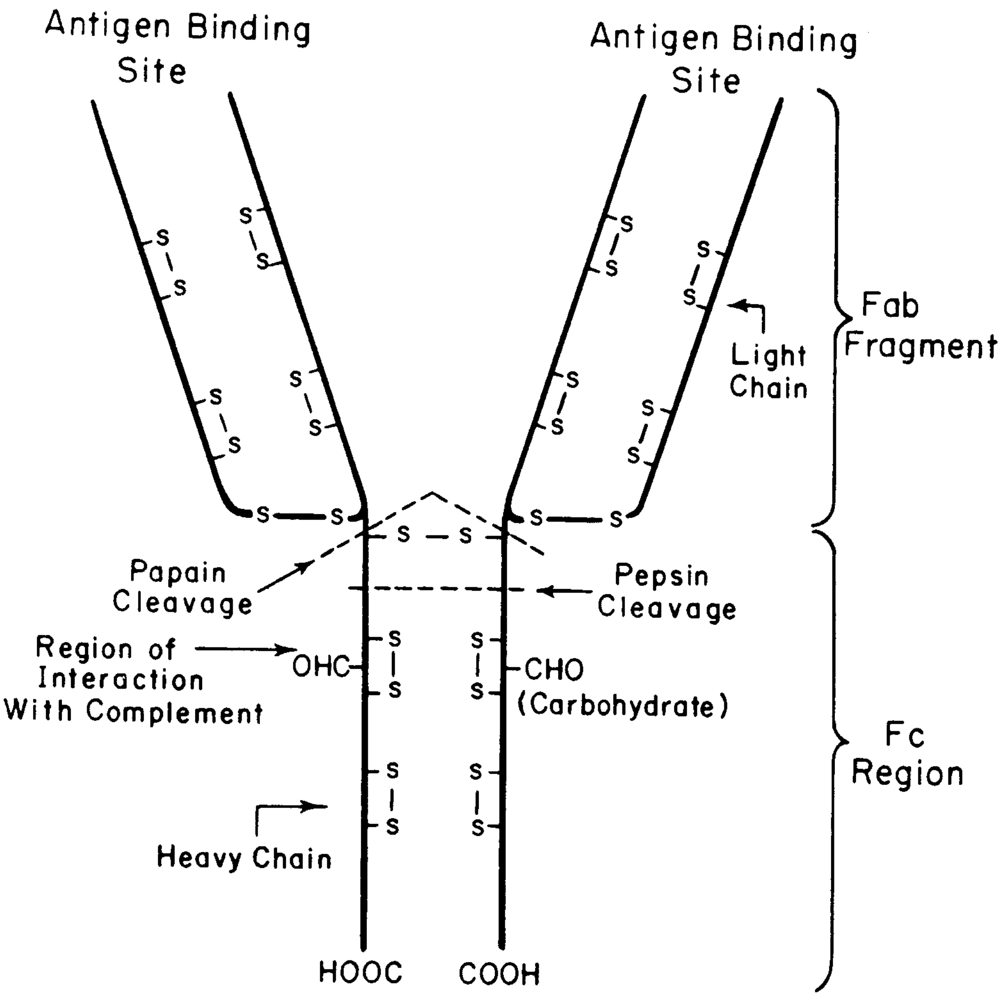
Antigen Binding Site
Antigen Binding Site
Fab Fragment
Light Chain
Papain Cleavage
Pepsin Cleavage
Region of Interaction With Complement
OHC
CHO (Carbohydrate)
Fc Region
Heavy Chain
HOOC
COOH

FIG. 1b

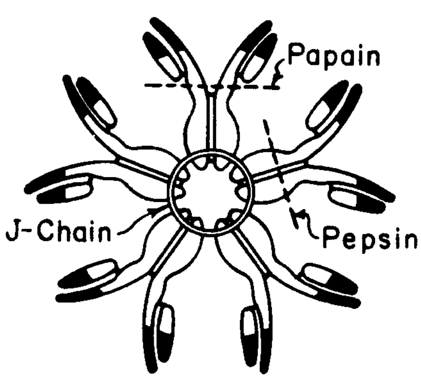
Papain
J-Chain
Pepsin

FIG. 2

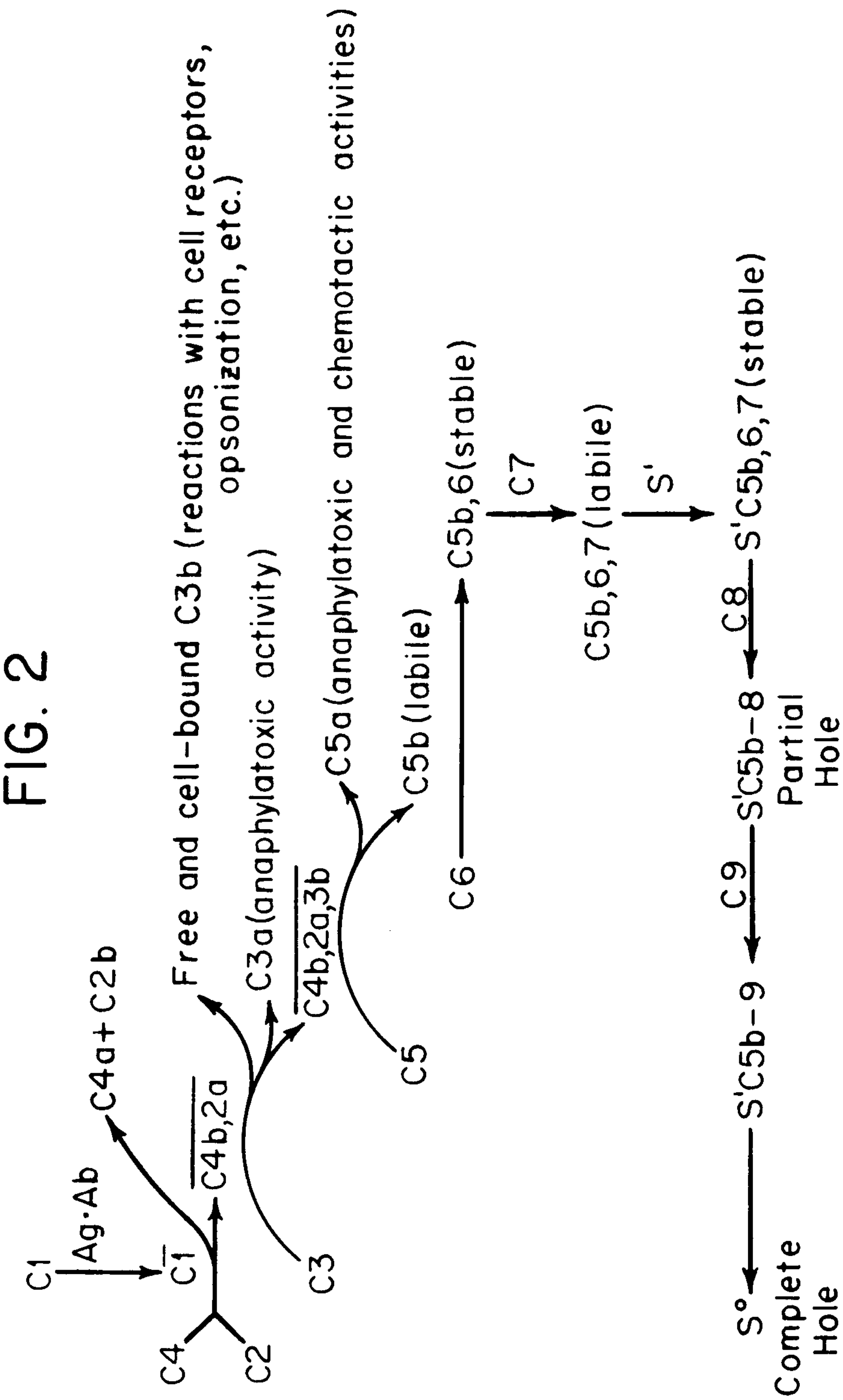
C1
Ag·Ab
C1
C4
C2
C4a+C2b
C4b,2a
C3
Free and cell-bound C3b (reactions with cell receptors, opsonization, etc.)
C3a (anaphylatoxic activity)
C4b,2a,3b
C5
C5a (anaphylatoxic and chemotactic activities)
C5b (labile)
C6
C5b,6 (stable)
C7
C5b,6,7 (labile)
S'
S'C5b,6,7 (stable)
C8
S'C5b-8
Partial Hole
C9
S'C5b-9
S°
Complete Hole

FIG. 3

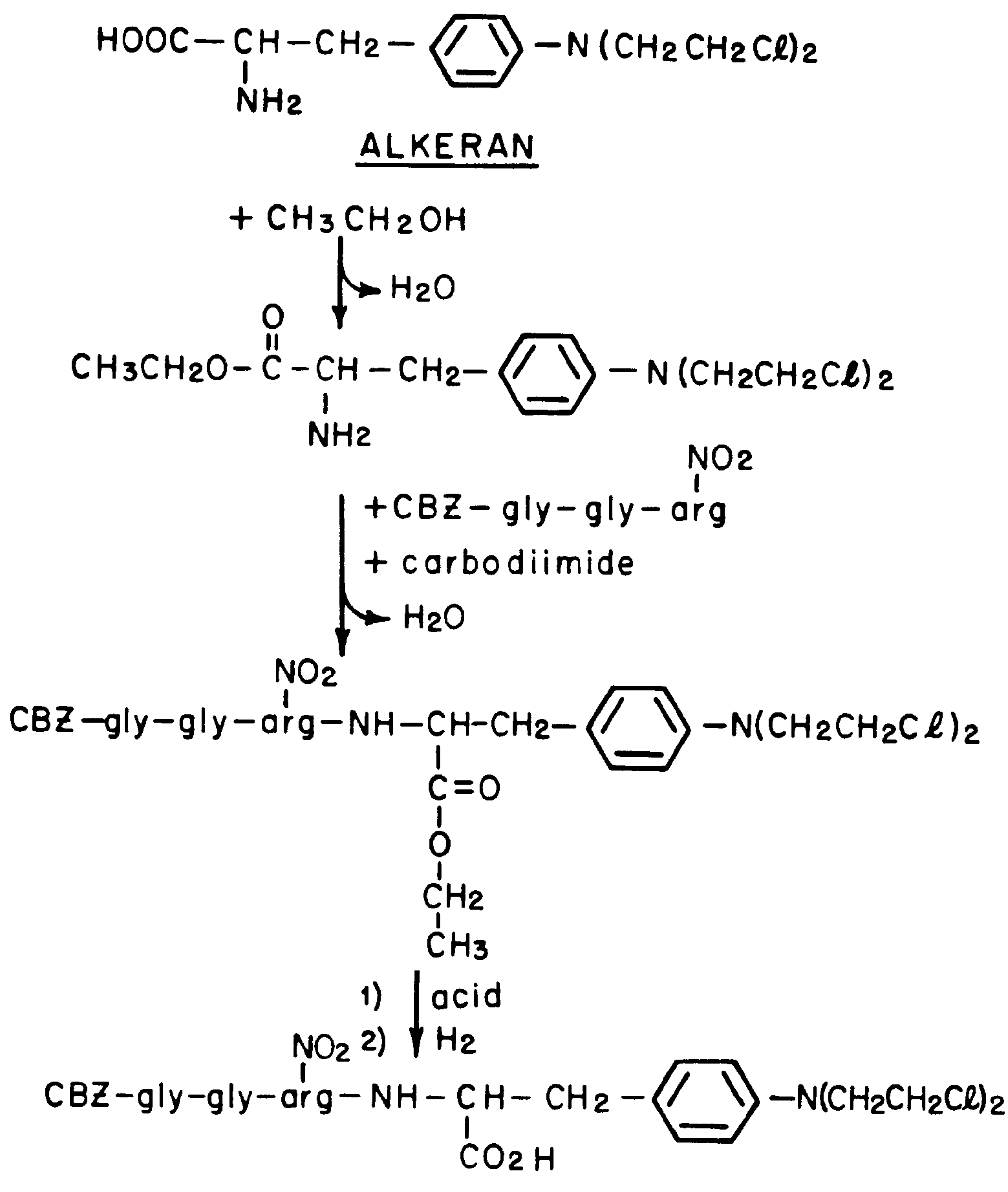


TRIPEPTIDE – ALKERAN

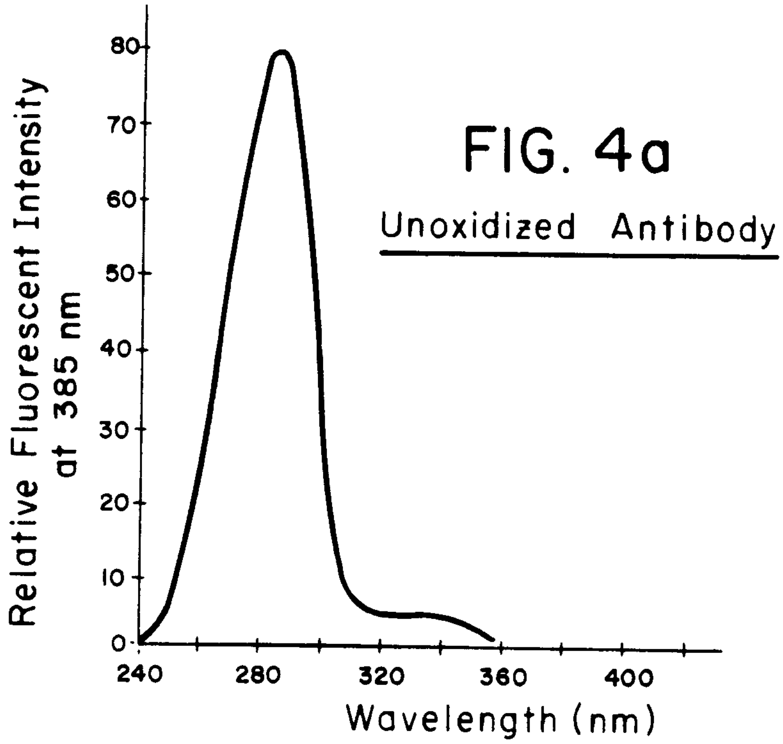


FIG. 4a

Unoxidized Antibody

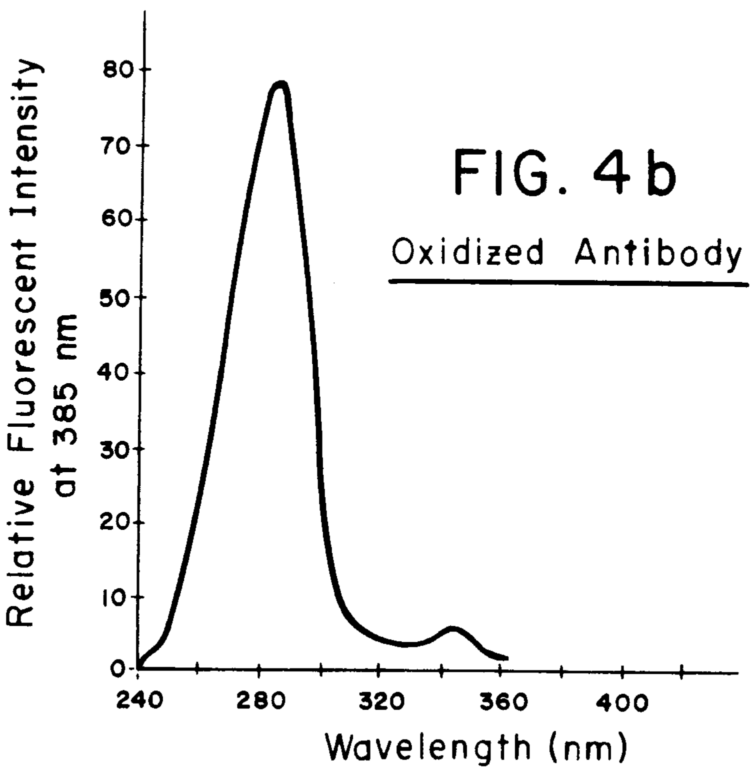


FIG. 4b

Oxidized Antibody

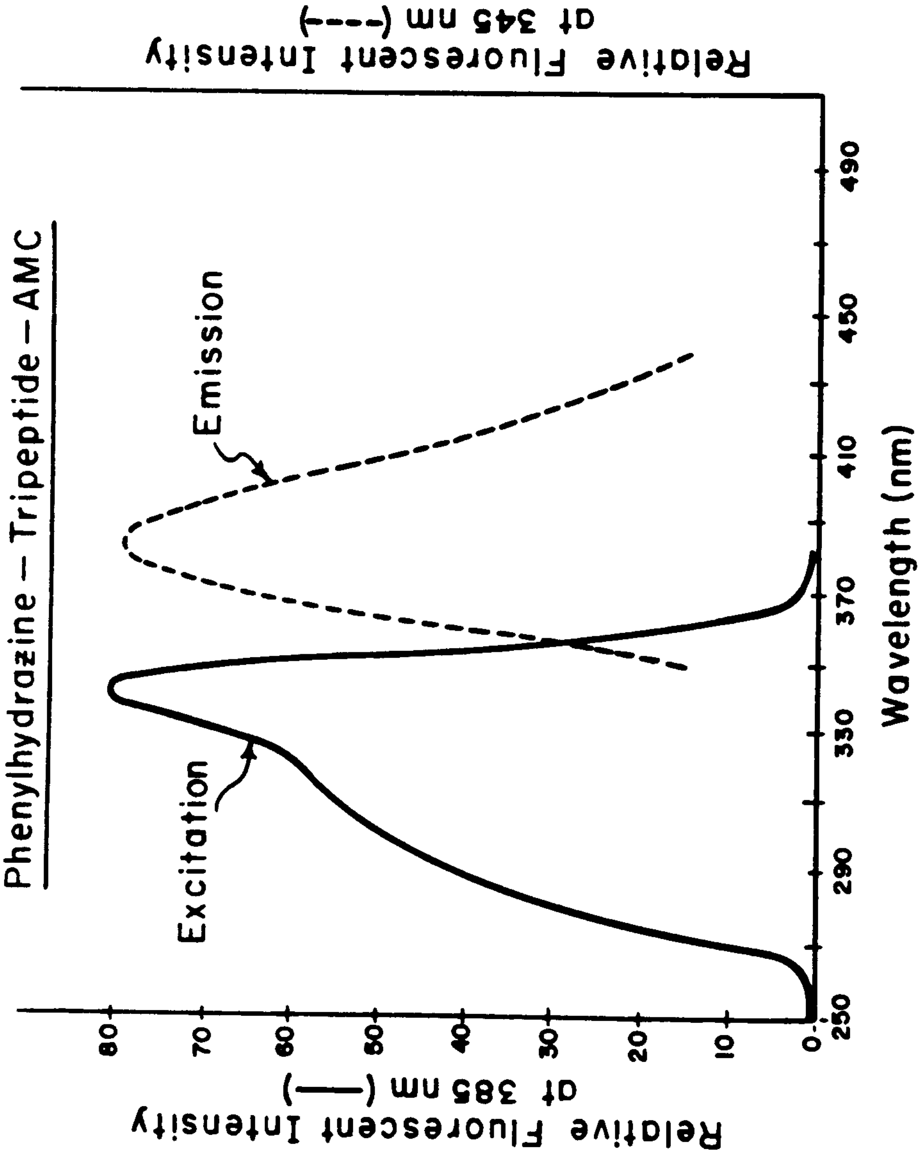
Phenylhydrazine – Tripeptide – AMC
Emission
Excitation
Relative Fluorescent Intensity at 385 nm (——)
0
10
20
30
40
50
60
70
80
Relative Fluorescent Intensity at 345 nm (----)
250
290
330
370
410
450
490
Wavelength (nm)

FIG. 5

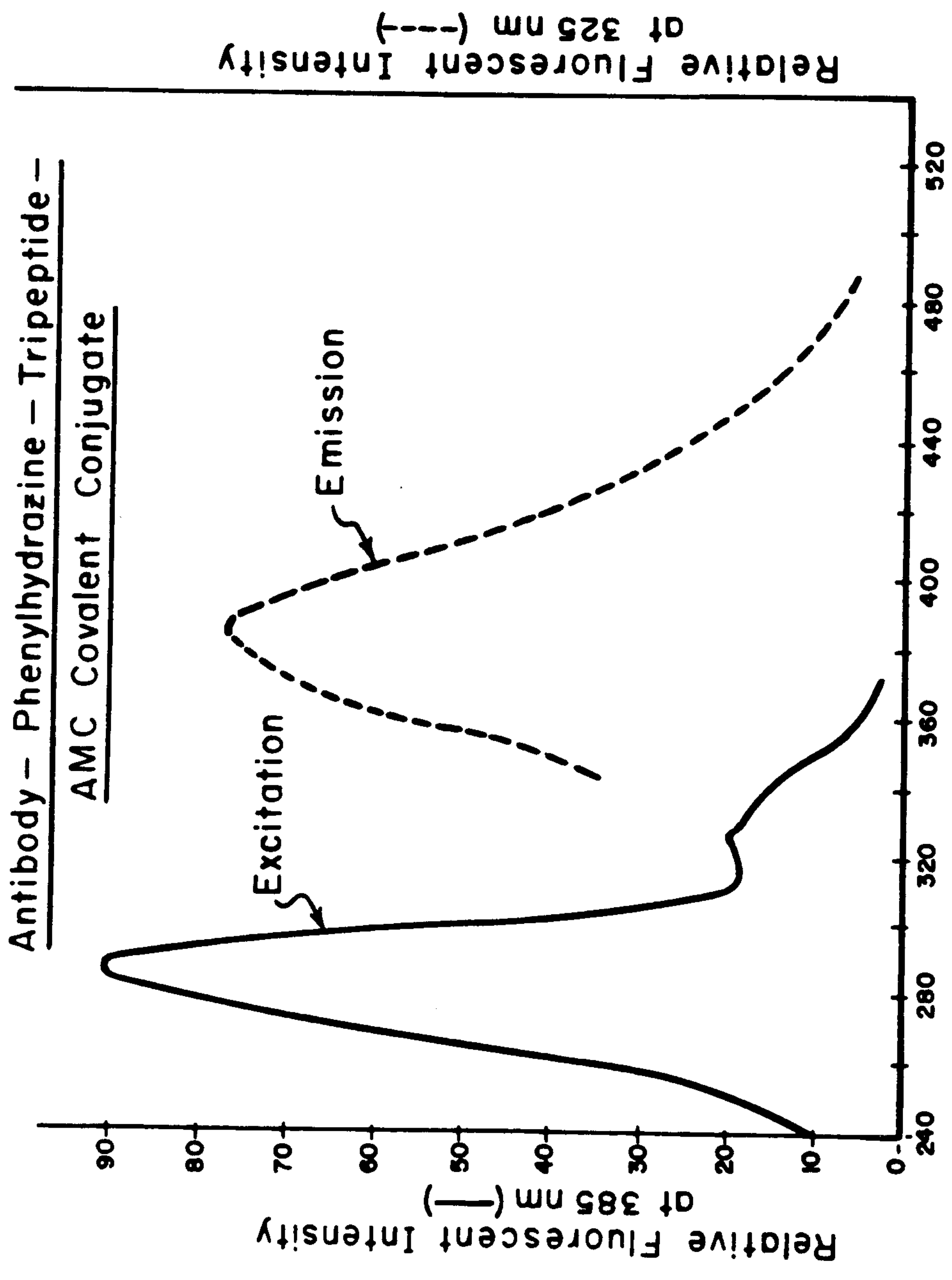
Antibody – Phenylhydrazine – Tripeptide – AMC Covalent Conjugate
Excitation
Emission
Relative Fluorescent Intensity at 385 nm (——)
Relative Fluorescent Intensity at 325 nm (----)
Wavelength (nm)
0
10
20
30
40
50
60
70
80
90
240
280
320
360
400
440
480
520

FIG. 6

FIG. 7

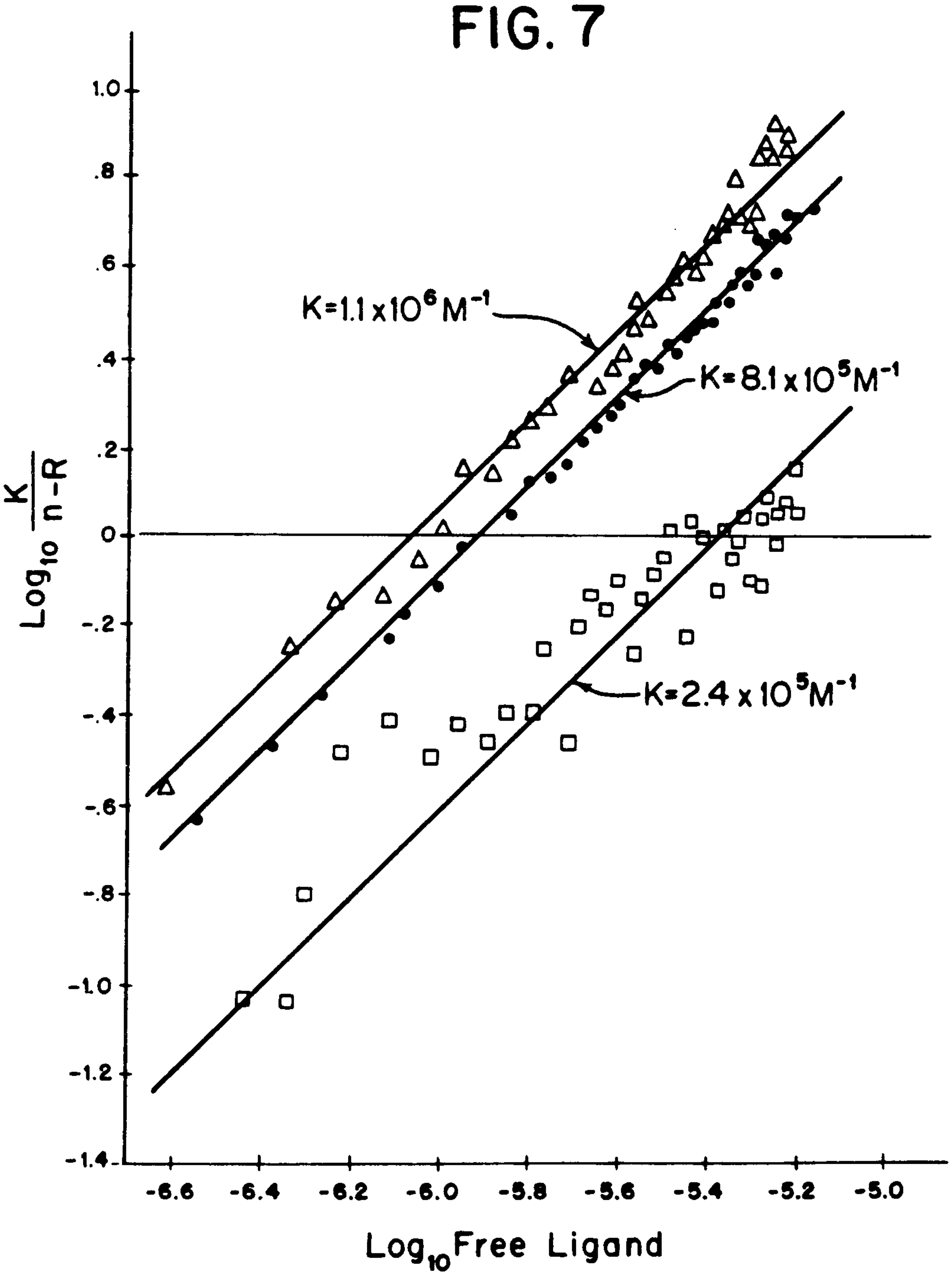
1.0
.8
.6
.4
.2
0
-.2
-.4
-.6
-.8
-1.0
-1.2
-1.4
Log10 K/(n-R)
K=1.1x10⁶M⁻¹
K=8.1x10⁵M⁻¹
K=2.4x10⁵M⁻¹
-6.6
-6.4
-6.2
-6.0
-5.8
-5.6
-5.4
-5.2
-5.0
Log10 Free Ligand

FIG. 8

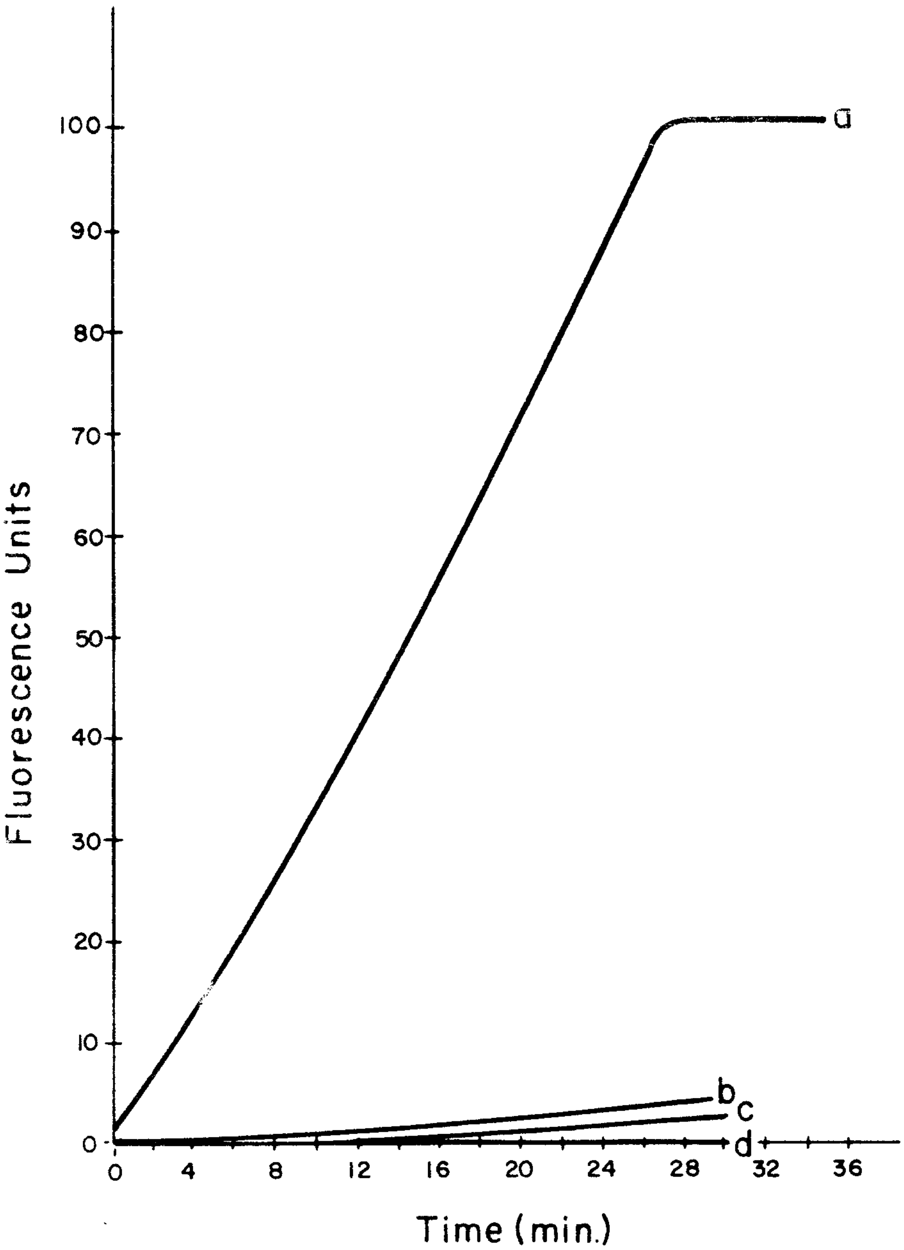
Fluorescence Units
100
90
80
70
60
50
40
30
20
10
0
0
4
8
12
16
20
24
28
32
36
Time (min.)
a
b
c
d